(19) Europäisches Patentamt
European Patent Office
Office européen des brevets

(11) Veröffentlichungsnummer : **0 457 727 A1**

# (12) EUROPÄISCHE PATENTANMELDUNG

(21) Anmeldenummer : **91810355.7**

(51) Int. Cl.[5] : **C07D 239/42,** A01N 43/54

(22) Anmeldetag : **08.05.91**

(30) Priorität : **17.05.90 CH 1667/90**

(43) Veröffentlichungstag der Anmeldung :
**21.11.91 Patentblatt 91/47**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Hubele, Adolf, Dr.**
**Obere Egg 9**
**CH-4312 Magden (CH)**

## (54) Schädlingsbekämpfungsmittel.

(57) Verbindungen der Formel

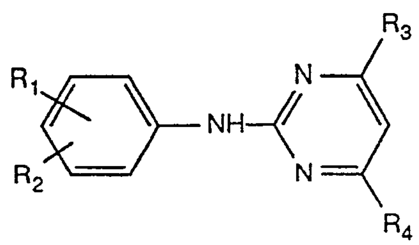

,

in welcher bedeuten :
$R_1$ und $R_2$ unabhängig voneinander Wasserstoff, Halogen, $C_1$-$C_3$-Alkyl, $C_1$-$C_2$-Halogenalkyl, $C_1$-$C_3$-Alkoxy oder $C_1$-$C_3$ Halogenalkoxy ;
$R_3$ $C_1$-$C_4$-Alkyl, durch Halogen oder Hydroxy substituiertes $C_1$-$C_2$-Alkyl, $C_3$-$C_6$-Cycloalkyl oder durch Methyl oder Halogen bis zu dreifach gleich oder verschieden substituiertes $C_3$-$C_6$-Cycloalkyl ;
$R_4$ die Reste $CH{=}NOR_5$, $CH{=}NR_6$ oder $CH_2NHR_6$ ;
$R_5$ Wasserstoff, $C_1$-$C_8$-Alkyl oder $C_1$-$C_4$-Alkyl, das durch Halogen, Hydroxy, Cyano, $C_1$-$C_4$-Alkoxy, $COOC_1$-$C_3$-Alkyl oder durch Phenyl substituiert ist, wobei das Phenyl durc Halogen, $C_1$-$C_3$-Alkyl, $C_1$-$C_3$-Alkoxyl oder Nitro substituiert sein kann ;
$R_5$ ferner $C_3$-$C_6$-Alkenyl oder durch Halogen substituiertes $C_3$-$C_6$-Alkenyl ; $C_3$-$C_6$-Alkinyl oder durch Halogen substituiertes $C_3$-$C_6$-Alkinyl, oder einen Acylrest COR′, worin R′ $C_1$-$C_6$-Alkyl oder durch Halogen, $C_1$-$C_3$-Alkoxy substituiertes $C_1$-$C_6$-Alkyl ; $C_3$-$C_6$-Alkenyl oder durch Halogen substituiertes $C_3$-$C_6$-Alkenyl ; Phenyl oder durch Halogen, $C_1$-$C_3$-Alkyl, $C_1$-$C_3$-Alkoxy oder Nitro substituiertes Phenyl bedeutet ;
$R_5$ ferner einen Carbamoylrest CO-N(R‴)R″ oder einen Oxycarbonylrest COOR″, worin R″ einen aliphatischen oder cycloaliphatischen Rest mit maximal 6 C-Atomen darstellt, der unsubstituiert oder durch Halogen substituiert ist, oder worin R″ einen Phenyl- oder Benzylrest bedeutet, der unsubstituiert oder im Ring durch Halogen, $C_1$-$C_3$-Alkyl, $C_1$-$C_3$-Alkoxy oder Nitro substituiert ist ; und worin R‴ Wasserstoff oder $C_1$-$C_4$-Alkyl bedeutet ;
$R_6$ Wasserstoff, $C_1$-$C_8$-Alkyl oder $C_1$-$C_4$-Alkyl, das durch Halogen, Hydroxy, Cyano, $C_1$-$C_4$-Alkoxy, $C_1$-$C_4$-Alkylamino, Bis-($C_1$-$C_4$-Alkyl)-amino, $C_3$-$C_6$-Cycloalkyl, $COOC_1$-$C_3$-Alkyl oder durch Phenyl substituiert ist, wobei das Phenyl durch Halogen, $C_1$-$C_3$-Alkyl, $C_1$-$C_3$-Alkoxy oder Nitro substituiert sein kann ;
$R_6$ ferner $C_3$-$C_6$-Cycloalkyl oder durch Methyl substituiertes $C_3$-$C_6$-Cycloalkyl ; $C_3$-$C_6$-Alkenyl oder durch Halogen substituiertes $C_3$-$C_6$-Alkenyl ; $C_3$-$C_6$-Alkinyl oder durch Halogen substituiertes $C_3$-$C_6$-Alkinyl ;
$R_6$ ferner Phenyl oder durch Halogen, $C_1$-$C_3$-Alkyl, $C_1$-$C_3$-Alkoxy, $C_1$-$C_2$-Halogenalkyl, $C_1$-$C_2$-Halogenalkoxy oder Nitro substituiertes Phenyl ; einen 5- oder 6-gliedrigen gesättigten oder ungesättigten heterocyclischen Rest mit ein bis 3 Heteroatomen, N, O oder S, wobei der heterocyclische Rest über ein $CH_2$-Brückenglied gebunden und durch Halogen, $C_1$-$C_3$-Alkyl, $C_1$-$C_3$-Alkoxy oder Nitro substituiert sein kann ;
$R_6$ ferner die Gruppe $NH$-$CO$-$NH_2$, $NH$-$CS$-$NH_2$ oder $N(R_7)R_8$, worin $R_7$ Wasserstoff oder $C_1$-$C_3$-Alkyl und $R_8$ Wasserstoff, $C_1$-$C_3$-Alkyl, Phenyl oder durch Halogen, $C_1$-$C_3$-Alkyl, $C_1$-$C_3$-Alkoxy oder Nitro substituiertes Phenyl darstellen ; unter Einschluss ihrer Säureadditionssalze oder Metallsalzkomplexe.
Die neuen Wirkstoffe besitzen wertvolle mikrobizide Eigenschaften. Sie können im Pflanzenschutz

EP 0 457 727 A1

Jouve, 18, rue Saint-Denis, 75001 PARIS

zur Verhütung des Befalls von Kulturpflanzen durch phytopathogene Mikroorganismen und zur Bekämpfung dieser Schädlinge eingesetzt werden.

Die vorliegende Erfindung betrifft neue 2-Anilino-pyrimidin-Derivate der nachstehenden Formel 1. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem von pflanzenschädigenden Mikroorganismen, vorzugsweise Pilzen.

Die erfindungsgemässen Verbindungen entsprechen der allgemeinen Formel I

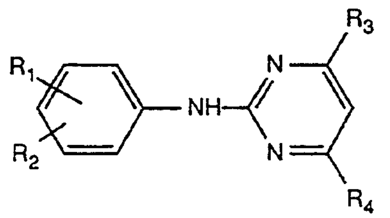

(I)

in welcher bedeuten:

$R_1$ und $R_2$ unabhängig voneinander Wasserstoff, Halogen, $C_1$-$C_3$-Alkyl, $C_1$-$C_2$-Halogenalkyl, $C_1$-$C_3$-Alkoxy oder $C_1$-$C_3$ Halogenalkoxy;

$R_3$ $C_1$-$C_4$-Alkyl, durch Halogen oder Hydroxy substituiertes $C_1$-$C_2$-Alkyl, $C_3$-$C_6$-Cycloalkyl oder durch Methyl oder Halogen bis zu dreifach gleich oder verschieden substituiertes $C_3$-$C_6$-Cycloalkyl;

$R_4$ die Reste $CH{=}NOR_5$, $CH{=}NR_6$ oder $CH_2NHR_6$;

$R_5$ Wasserstoff, $C_1$-$C_8$-Alkyl oder $C_1$-$C_4$-Alkyl, das durch Halogen, Hydroxy, Cyano, $C_1$-$C_4$-Alkoxy, $COOC_1$-$C_3$-Alkyl oder durch Phenyl substituiert ist, wobei das Phenyl durc Halogen, $C_1$-$C_3$-Alkyl, $C_1$-$C_3$-Alkoxyl oder Nitro substituiert sein kann;

$R_5$ ferner $C_3$-$C_6$-Alkenyl oder durch Halogen substituiertes $C_3$-$C_6$-Alkenyl; $C_3$-$C_6$-Alkinyl oder durch Halogen substituiertes $C_3$-$C_6$-Alkinyl, oder einen Acylrest COR′, worin R′ $C_1$-$C_6$-Alkyl oder durch Halogen, $C_1$-$C_3$-Alkoxy substituiertes $C_1$-$C_6$-Alkyl; $C_3$-$C_6$-Alkenyl oder durch Halogen substituiertes $C_3$-$C_6$-Alkenyl; Phenyl oder durch Halogen, $C_1$-$C_3$-Alkyl, $C_1$-$C_3$-Alkoxy oder Nitro substituiertes Phenyl bedeutet;

$R_5$ ferner einen Carbamoylrest CO-N(R‴)R″ oder einen Oxycarbonylrest COOR″, worin R″ einen aliphatischen oder cycloaliphatischen Rest mit maximal 6 C-Atomen darstellt, der unsubstituiert oder durch Halogen substituiert ist, oder worin R″ einen Phenyl- oder Benzylrest bedeutet, der unsubstituiert oder im Ring durch Halogen, $C_1$-$C_3$-Alkyl, $C_1$-$C_3$-Alkoxy oder Nitro substituiert ist; und worin R‴ Wasserstoff oder $C_1$-$C_4$-Alkyl bedeutet;

$R_6$ Wasserstoff, $C_1$-$C_8$-Alkyl oder $C_1$-$C_4$-Alkyl, das durch Halogen, Hydroxy, Cyano, $C_1$-$C_4$-Alkoxy, $C_1$-$C_4$-Alkylamino, Bis-($C_1$-$C_4$-Alkyl)-amino, $C_3$-$C_6$-Cycloalkyl, $COOC_1$-$C_3$-Alkyl oder durch Phenyl substituiert ist, wobei das Phenyl durch Halogen, $C_1$-$C_3$-Alkyl, $C_1$-$C_3$-Alkoxy oder Nitro substituiert sein kann;

$R_6$ ferner $C_3$-$C_6$-Cycloalkyl oder durch Methyl substituiertes $C_3$-$C_6$-Cycloalkyl; $C_3$-$C_6$-Alkenyl oder durch Halogen substituiertes $C_3$-$C_6$-Alkenyl; $C_3$-$C_6$-Alkinyl oder durch Halogen substituiertes $C_3$-$C_6$-Alkinyl;

$R_6$ ferner Phenyl oder durch Halogen, $C_1$-$C_3$-Alkyl, $C_1$-$C_3$-Alkoxy, $C_1$-$C_2$-Halogenalkyl, $C_1$-$C_2$-Halogenalkoxy oder Nitro substituiertes Phenyl; einen 5- oder 6-gliedrigen gesättigten oder ungesättigten heterocyclischen Rest mit ein bis 3 Heteroatomen, N, O oder S, wobei der heterocyclische Rest über ein $CH_2$-Brückenglied gebunden und durch Halogen, $C_1$-$C_3$-Alkyl, $C_1$-$C_3$-Alkoxy oder Nitro substituiert sein kann;

$R_6$ ferner die Gruppe $NH$-$CO$-$NH_2$, $NH$-$CS$-$NH_2$ oder $N(R_7)R_8$, worin $R_7$ Wasserstoff oder $C_1$-$C_3$-Alkyl und $R_8$ Wasserstoff, $C_1$-$C_3$-Alkyl, Phenyl oder durch Halogen, $C_1$-$C_3$-Alkyl, $C_1$-$C_3$-Alkoxy oder Nitro substituiertes Phenyl darstellen; unter Einschluss ihrer Säureadditionssalze oder Metallsalzkomplexe.

Unter Alkyl selbst oder als Bestandteil eines anderen Substituenten, wie Halogenalkyl, Alkoxy oder Halogenalkoxy, sind je nach Anzahl der benannten Kohlenstoffatome beispielsweise zu verstehen: Methyl, Ethyl, Propyl, Butyl, Pentyl, Hexyl, Heptyl, Octyl sowie ihre Isomeren, wie z.B. Isopropyl, Isobutyl, tert.-Butyl, Isopentyl usw.. Halogen steht für Fluor, Chlor, Brom oder Jod. Halogenalkyl und Halogenalkoxy bezeichnen einfach bis perhalogenierte Reste, wie z.B. $CHCl_2$, $CH_2F$, $CCl_3$, $CH_2Cl$, $CHF_2$, $CH_2CH_2Br$, $C_2Cl_5$, $CH_2Br$, $CHBrCl$ usw., vorzugsweise $CF_3$. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl. Cycloalkyl steht je nach Zahl der genannten Kohlenstoffatome z.B. für Cyclopropyl, Cyclobutyl, Cyclopentyl oder Cyclohexyl.

Als Heterocyclen sind anzusehen z.B.: Furan, Thiophen, Pyridin, Pyrimidin, Thiazol oder Pyridazin. Insbesondere stellen sie dar: Furan-2-yl, Thiophen-2-yl, Pyridin-2-yl, Pyridin-3-yl, Pyridin-4-yl, Pyrimidin-2-yl, Pyrimidin-4-yl, Pyrimidin-5-yl, Thiazol-2-yl, Thiazol-4-yl oder Pyridazin-2-yl.

Die Verbindungen der Formel I sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende fungizide Wirkung, sondern auch durch besonders gute Pflanzenverträglichkeit aus.

Die Erfindung betrifft sowohl die freien Verbindungen der Formel I als auch deren Additionssalze mit anorganischen und organischen Säuren sowie deren Komplexe mit Metallsalzen.

Erfindungsgemässe Salze sind insbesondere Additionssalze mit unbedenklichen anorganischen oder organischen Säuren, beispielsweise Halogenwasserstoffsäuren, z.B. Chlor-, Brom- oder Jodwasserstoffsäure, Schwefelsäure, Phosphorsäure, phosphorige Säure, Salpetersäure, oder organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicyclsäure, 2-Phenoxybenzoesäure, 2-Acetoxybenzoesäure oder 1,2-Naphthalin-disulfonsäure.

Metallsalzkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicyclaten, Benzoaten usw. der Elemente der zweiten Hauptgruppe wie Calcium und Magnesium und der dritten und vierten Hauptgruppe wie Aluminium, Zinn oder Blei sowie der ersten bis achten Nebengruppe wie Chrom, Mangan, Eisen, Kobalt, Nickel, Kupfer, Zink usw.. Bevorzugt sind die Nebengruppen-Elemente der 4. Periode. Die Metalle können dabei in den verschiedenen ihnen zukommenden Wertigkeiten vorliegen. Die Metallkomplexe können ein- oder mehrkernig auftreten, d.h. sie können ein oder mehrere organische Molekülanteile als Liganden enthalten.

Folgende Wirkstoffgruppen sind aufgrund ihrer ausgeprägten mikrobiziden, insbesondere pflanzenfungiziden, Aktivität bevorzugt:

1) Verbindungen der Formel I, worin bedeuten:

$R_1$ und $R_2$ unabhängig voneinander Wasserstoff, Halogen oder Methyl;

$R_3$ Methyl, Ethyl, Cyclopropyl oder durch Methyl oder Halogen ein- oder zweifach substituiertes Cyclopropyl;

$R_5$ Wasserstoff, $C_1$-$C_4$-Alkyl, durch Halogen, Hydroxy oder $C_1$-$C_2$-Alkoxy substituiertes $C_1$-$C_2$-Alkyl, Benzyl oder durch Halogen, Methyl oder Methoxy substituiertes Benzyl, $C_3$-$C_4$-Alkenyl, $C_3$-$C_4$-Alkinyl oder durch Halogen substituiertes $C_3$-$C_4$-Alkinyl;

$R_5$ ferner COR′, worin R′ $C_1$-$C_3$-Alkyl oder durch Halogen oder $C_1$-$C_2$-Alkoxy substituiertes $C_1$-$C_3$-Alkyl, Phenyl oder durch Halogen, Methyl oder Methoxy substituiertes Phenyl darstellt; oder COOR″, worin R″ $C_1$-$C_4$-Alkyl oder Phenyl bedeutet; oder CONHR″, worin R″ $C_1$-$C_4$-Alkyl oder Phenyl oder durch Halogen substituiertes Halogen darstellt;

$R_5$ ferner die Reste $CON(CH_3)_2$ oder $CON(CH_3)OCH_3$;

$R_6$ Wasserstoff, $C_1$-$C_4$-Alkyl, durch Halogen, Hydroxy, $C_1$-$C_2$-Alkoxy, $C_1$-$C_2$-Alkylamino, Bis-($C_1$-$C_2$-Alkyl)-amino, Cyclopropyl, Cyclohexyl, Benzyl oder p-Chlorbenzyl substituiertes $C_1$-$C_2$-Alkyl;

$R_6$ ferner Cyclopropyl, Cyclohexyl, Allyl, Chlorallyl oder Propargyl;

$R_6$ ferner Phenyl, durch Halogen, Methyl, Methoxy, Trifluormethyl oder Difluormethoxy substituiertes Phenyl; einen 5- oder 6-gliedrigen gesättigten oder ungesättigten heterocyclischen Rest mit ein bis drei Heteroatomen, N, O oder S, wobei der heterocyclische Rest über ein $CH_2$-Brückenglied gebunden und durch Halogen, Methyl, Methoxy oder Nitro substituiert sein kann;

$R_6$ ferner die Gruppe $NH-CO-NH_2$, $NH-CS-NH_2$ oder $N(R_7)R_8$, worin $R_7$ Wasserstoff oder Methyl und $R_8$ Wasserstoff, Methyl, Phenyl oder durch Halogen, Methyl, Methoxy substituiertes Phenyl bedeuten.

2) Verbindungen der Formel I, worin bedeuten:

$R_1$ und $R_2$ unabhängig voneinander Wasserstoff oder Fluor;

$R_3$ Methyl, Ethyl, Cyclopropyl oder durch Methyl substituiertes Cyclopropyl;

$R_5$ Wasserstoff, Methyl, Ethyl, Hydroxyethyl, Halogenethyl, Benzyl oder durch Fluor, Chlor oder Methyl substituiertes Benzyl, $C_3$-$C_4$-Alkenyl, $C_3$-$C_4$-Alkinyl oder durch Halogen substituiertes $C_3$-$C_4$-Alkinyl;

$R_6$ ferner COR′, worin R′Methyl oder Ethyl oder durch Fluor, Chlor oder Brom substituiertes Methyl oder Ethyl; Phenyl oder durch Fluor, Chlor oder Methyl substituiertes Phenyl darstellt; oder COOR″, worin R″ Methyl, Ethyl oder CONHR″, worin R″ Methyl, Ethyl, Phenyl oder durch Chlor substituiertes Phenyl darstellt;

$R_5$ ferner die Reste $CON(CH_3)_2$ oder $CON(CH_3)OCH_3$;

$R_6$ Wasserstoff, Methyl, Ethyl, durch Chlor, Hydroxy, Methoxy, Methylamino, Dimethylamino, Cyclopropyl, Benzyl oder p-Chlorbenzyl substituiertes $C_1$-$C_2$-Alkyl;

$R_6$ ferner Cyclopropyl, Allyl, Chlorallyl oder Propargyl;

$R_6$ ferner Phenyl, durch Halogen, Methyl, Methoxy, Trifluormethyl oder Difluormethoxy substituiertes Phenyl; einen heterocyclischen Rest, z.B. Furan-2-yl, Thiophen-2-yl, Thiazol-2-yl, Pyridin-2-yl, Pyridin-3-yl, Pyridin-4-yl, Pyrimidin-2-yl, Pyrimidin-4-yl oder Pyrimidin-5-yl.

$R_6$ ferner die Gruppe NH-CO-$NH_2$ oder $N(R_7)R_8$, worin $R_7$ Wasserstoff oder Methyl und $R_8$ Wasserstoff, Methyl, Phenyl oder durch Chlor oder Methyl substituiertes Phenyl bedeuten.

3) Verbindungen der Formel I, worin bedeuten:

$R_1$ und $R_2$ Wasserstoff;

$R_3$ Methyl, Ethyl oder Cyclopropyl;

$R_5$ Wasserstoff, Methyl, Ethyl, Hydroxyethyl, Bromethyl, Chlorethyl, Benzyl oder durch Fluor, Chlor oder Methyl substituiertes Benzyl, Allyl, Propargyl oder Jodpropargyl;

$R_5$ ferner COR', worin R'Methyl oder durch Chlor oder Brom substituiertes Methyl; Phenyl oder durch Fluor, Chlor oder Methyl substituiertes Phenyl darstellt; oder COOR", oder CONHR", worin R" Methyl, Ethyl oder Phenyl bedeutet;

$R_6$ Wasserstoff, Methyl, Ethyl, durch Chlor, Hydroxy, Cyclopropyl oder Benzyl substituiertes $C_1$-$C_2$-Alkyl; oder Cyclopropyl, Cyclohexyl, Allyl oder Propargyl;

$R_6$ ferner Phenyl oder durch Fluor Chlor, Methyl oder Trifluormethyl substituiertes Phenyl; einen heterocyclischen Rest, z.B. Thiophen-2-yl, Thiazol-2-yl, Pyridin-2-yl, Pyridin-3-yl oder Pyrimidin-2-yl;

$R_6$ ferner die Gruppe NH-CO-$NH_2$ oder $NHR_8$, worin $R_8$ Wasserstoff, Methyl, Phenyl oder durch Chlor oder Methyl substituiertes Phenyl bedeuten.

Als besonders bevorzugte Einzelverbindungen sind folgende zu nennen:

2-Anilino-4-formyloxim-6-methyl-pyrimidin (Verb. Nr. 1.1 );
2-Anilino-4-formyl-O-methyloxim-6-methyl-pyrimidin (Verb. Nr. 1.4);
2-Anilino-4-formyloxim-6-ethyl-pyrimidin (Verb. Nr. 1.14);
2-Anilino-4-formyl-O-methyloxim-6-ethyl-pyrimidin (Verb. Nr. 1.45);
2-(3-Fluoranilino)-4-formyloxim-6-methyl-pyrimidin (Verb. Nr. 1.93);
2-(3-Fluoranilino)-4-formyl-O-methyloxim-6-methyl-pyrimidin (Verb. Nr. 1.89);
2-Anilino-4-formyl-(2-ethoxyethylimin)-6-cyclopropyl-pyrimidin (Verb. Nr. 2.1);
2-Anilino-4-formyl-(cyclopropylimin)-6-cyclopropyl-pyrimidin (Verb. Nr. 2.12).

Die Verbindungen der Formel I werden hergestellt, indem man

1.ein Guanidinsalz der Formel IIa

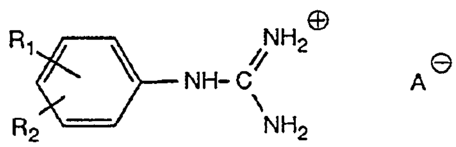

(IIa)

oder ein zugrundeliegendes Guanidin der Formel IIb

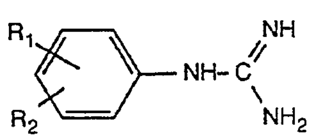

(IIb)

mit einem Diketon der Formel III,

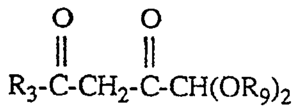

(III)

worin $R_9$ ein Alkyl, bevorzugt $C_1$-$C_4$-Alkyl, bedeutet, und $A^{\ominus}$ ein Säureanion darstellt, in einem protischen Lösungsmittel oder ohne Lösungsmittel bei Temperaturen von 40° bis 160°C, bevorzugt 60°C bis 110°C, zu einem Pyrimidinacetal der Formel IV

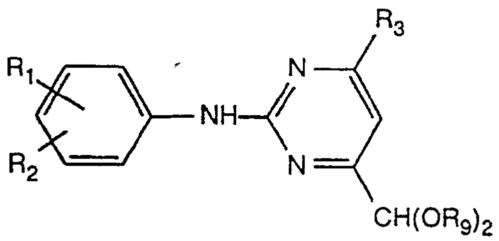

(IV)

umsetzt und das Acetal der Formel IV in Gegenwart einer Säure, z.B. Halogenwasserstoffsäure oder Schwefelsäure, in Wasser oder wässrigen Lösungsmittelgemischen, z.B. mit Lösungsmitteln wie Alkoholen oder Dimethylformamid, bei Temperaturen von 20° bis 100°C, bevorzugt 30°C bis 70°C, zu einem Pyrimidinaldehyd der Formel V

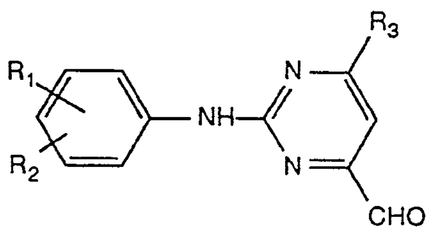

(V)

hydrolysiert und den Aldehyd der Formel V mit einem Hydroylaminsalz in Gegenwart einer Base in einem protischen Lösungsmittel, bevorzugt in Gegenwart von Wasser, bei Temperaturen von 10° bis 100°C, bevorzugt 10° bis 60°C, zu einem Oxim der Formel Ia

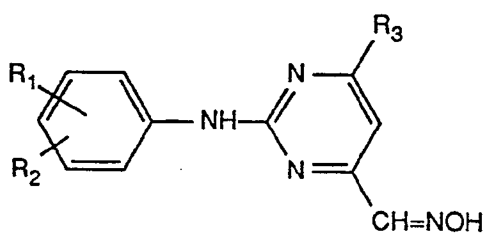

(Ia)

umsetzt, und das Oxim der Formel Ia mittels eines basischen Reagens, wie z.B Natriumhydrid oder Kaliumhydroxyd in ein Oximsalz der Formel Ib

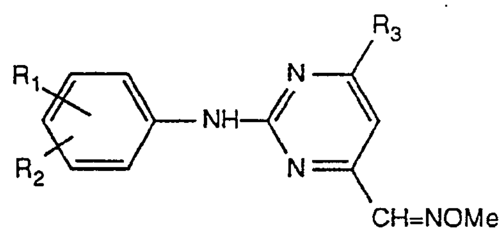

(Ib)

überführt, wobei Me ein Alkali oder Erdalkaliatom darstellt, und das Oximsalz der Formel (Ib) mit einer Verbindung der Formel VI

$R_5X$ (VI),

worin X eine nucleofuge Abgangsgruppe, wie z.B. Halogen, Mesylat, Tosylat u.a., vorzugsweise Chlor, Brom oder Jod, darstellt, in einem inerten Lösungsmittel bei Temperaturen von -30° bis 160°C, bevorzugt - 10° bis 110°C, zu einer Verbindung der Formel Ic

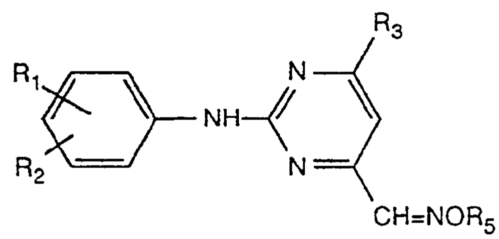

(Ic)

reagieren lässt; oder

2. ein Säurehalogenid der Formel VII, VIII oder IX

Hal-CO-R′ (VII)

Hal-CO-N(R″)R‴ (VIII)

Hal-CO-OR″ (IX)

oder ein analoges Säureanhydrid der Formel VII′, VIII′oder IX′

R′-CO-O-CO-R′ (VII′)

R‴(R″)N-CO-O-CO-N(R″)R‴ (VIII′)

R″O-CO-O-CO-OR″ (IX′)

mit einem Oxim der Formel Ia

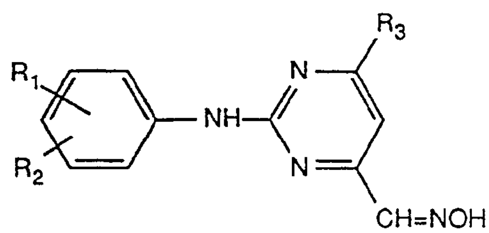

(Ia)

oder einem Salz davon in einem inerten Lösungsmittel und bei Verwendung der Säurehalogenide in Gegenwart von Protonenakzeptoren, z.B. tertiäre Amine, Alkali- oder Erdalkalihydroxide sowie Alkalihydride, bei Temperaturen von -20° bis 120°C, bevorzugt 0° bis 80°C, umsetzt, oder

3. ein Isocyanat der Formel X

R″N-CO (X)

mit einem Oxim der Formel Ia

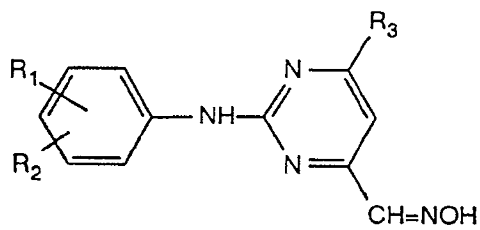

(Ia)

in einem inerten Lösungsmittel, wobei als Katalysator eine geringe Menge einer tertiären organischen Base, z.B. Triethylamin, zugegen sein kann, bei Temperaturen von 0° bis 120°C, bevorzugt 10° bis 80°C, umsetzt oder

4. eine Verbindung der Formel XI

$H_2N\text{-}OR_5 \cdot HA$ (XI)

mit einem Formyldiacetal der Formel IV

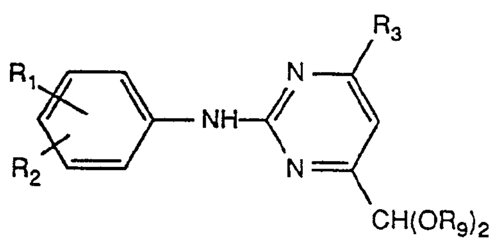

(IV)

oder einem Carbaldehyd der Formel V

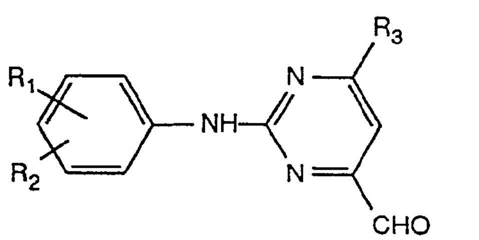

(V)

worin HA eine anorganische oder organische Säure, z.B. Schwefelsäure, Salzsäure, Essigsäure u.a., und $R_9$ ein Alkyl, bevorzugt $C_1$-$C_4$-Alkyl, darstellen, in einem inerten Lösungsmittel, z.B. einem protischen Lösungsmittel wie beispielsweise einem Alkohol, in Gegenwart einer Base (zur Freisetzung des Hydroxylamins) bei Temperaturen von 10° bis 110°C, bevorzugt 20° bis 80°C, umsetzt oder

5. ein Formyldiacetal der Formel IV

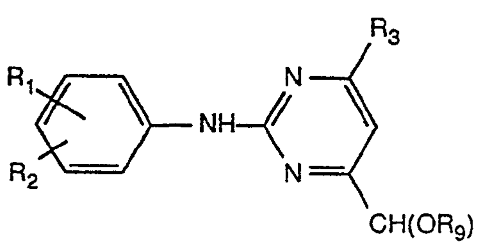

(IV)

oder ein Carbaldehyd der Formel V

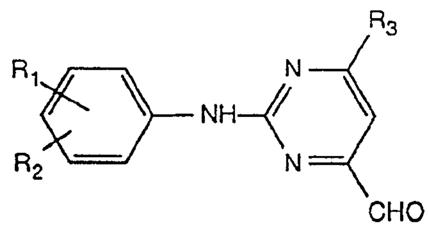

(V)

mit einem primären Amin der Formel XII

$H_2N\text{-}R_6$ (XII)

zu einer Verbindung der Formel Id

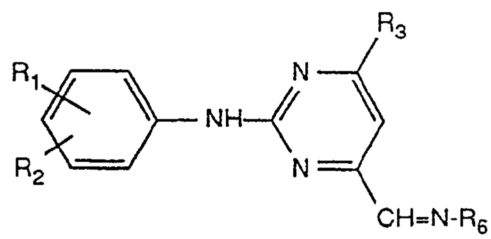

(Id)

worin $R_9$ ein Alkyl, bevorzugt $C_1$-$C_4$-Alkyl darstellt, in einem inerten Lösungsmittel, z.B. Benzol, Toluol, Cyclohexan u.a., vorzugsweise mit einem Lösungsmittel, das mit dem ausgeschiedenen Wasser ein Azeotrop bildet, in Gegenwart eines Katalysators (zur Beschleunigung der Wasserabspaltung), z.B. von geringen Mengen anorganischer oder organischer Säuren, z.B. p-Toluolsulfonsäure, bei Temperaturen von 60° bis 160°C, bevorzugt 80° bis 120°C, umsetzt oder

6. ein Hydrazinderivat der Formel XIII, XIV oder XV

$H_2N\text{-}N(R_7)R_8$ (XIII)

$H_2N\text{-}NH\text{-}CO\text{-}NH_2$ (XIV)

$H_2N\text{-}NH\text{-}CS\text{-}NH_2$ (XV)

mit einem Formyldiacetal der Formel IV

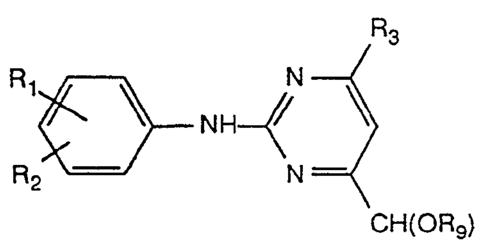

(IV)

oder einem Carbaldehyd der Formel V

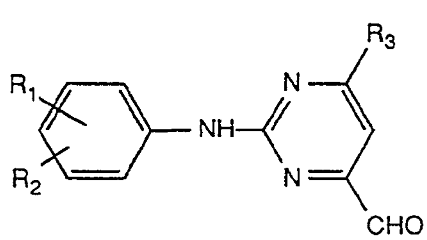

(V)

zu einer Verbindung der Formel Id'

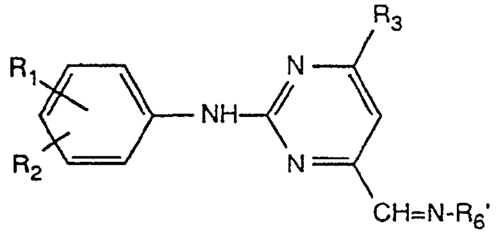

(Id')

worin $R_9$ ein Alkyl, bevorzugt $C_1$-$C_4$-Alkyl, und $R_6'$ die Reste NH-CO-$NH_2$, NH-CS-$NH_2$ oder $N(R_7)R_8$ darstellen, in einem inerten Lösungsmittel bei Temperaturen von -20° bis 120°C, bevorzugt 0° bis 80°C, umsetzt oder

7. eine Verbindung der Formel Id

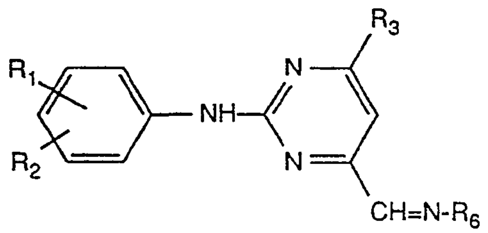

(Id)

durch Hydrierung mit elementarem Wasserstoff unter Normaldruck oder mit Ueberdruck in Gegenwart eines Katalysators, z.B. Palladium-Kohle oder Raney-Nickel in einem inerten Lösungsmittel, z.B. Ethylacetat, Tetrahydrofuran oder Dioxan, bei Temperaturen von 10° bis 120°C, bevorzugt 20° bis 60°C, in eine Verbindung der Formel Ie

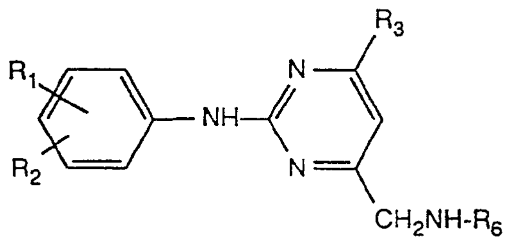

(Ie)

überführt; oder

8. eine Verbindung der Formel Ic

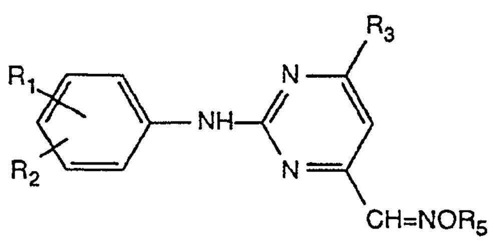

(Ic)

oder eine Verbindung der Formel Ig

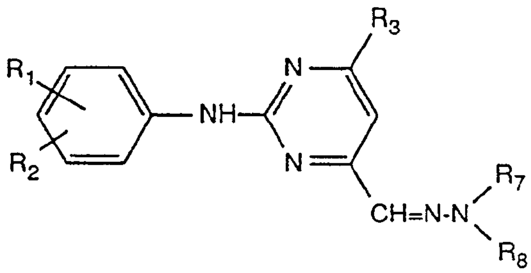

(Ig)

durch Hydrierung mit elementarem Wasserstoff unter Normaldruck oder mit Ueberdruck in Gegenwart eines Katalysators, z.B. Palladium-Kohle oder Raney-Nickel in einem inerten Lösungsmittel, z.B. Ethylacetat, Tetrahydrofuran oder Dioxan, bei Temperaturen von 10° bis 120°C, bevorzugt 20° bis 60°C, in eine Verbindung der Formel If

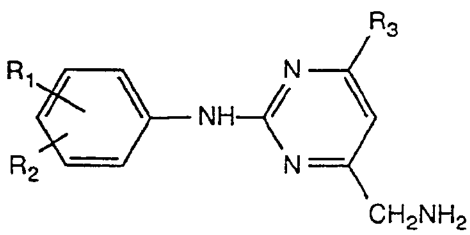

(If)

überführt.

In den vorstehenden Formeln Ia bis XVIII haben $R_1$ bis $R_8$ die unter Formel I angegebenen Bedeutungen.

Die als Zwischenprodukte zur Herstellung der neuen pflanzenmikrobiziden Wirkstoffe der Formel I verwendeten Acetalverbindungen der Formel IV können hergestellt werden, indem man

A) Hamstoff der Formel XVI

$$H_2N\text{-}CO\text{-}NH_2 \qquad (XVI)$$

mit einem Diketon der Formel III

$$R_3\text{-}CO\text{-}CH_2\text{-}CO\text{-}CH(OR_9)_2 \qquad (III)$$

in Gegenwart einer Säure in einem inerten Lösungsmittel bei Temperaturen von 20° bis 140°C, bevorzugt 20° bis 80°C, zu einer Pyrimidinverbindung der Formel XVII

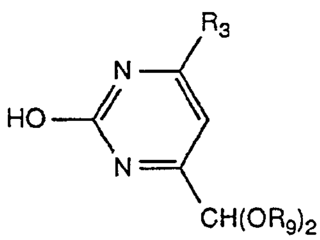

(XVII)

cyclisiert und die OH-Gruppe der erhaltenen Verbindung der Formel XVII weiter mit überschüssigem $PO(Hal)_3$ mit oder ohne Lösungsmittel bei Temperaturen von 50° bis 110°C, bevorzugt 50° bis 80°C, gegen ein Halogenatom austauscht und so eine Verbindung der Formel XVIII

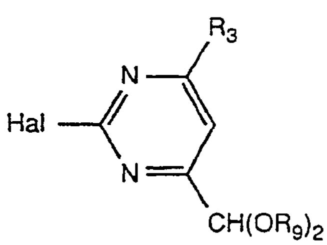

(XVIII)

wobei Hal Halogen, bevorzugt Chlor oder Brom, bedeutet, erhält und diese weiter mit einer Anilinverbin-

dung der Formel XIX

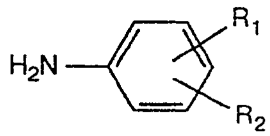

(XIX)

je nach Verfahrensbedingungen umsetzt, entweder

a) ohne Protonenakzeptor mit einer äquimolaren Menge oder einem geringen Uebeschuss der Verbindung der Formel XIX oder

b) in Gegenwart eines Protonenakzeptors, z.B. einem angemessenen Ueberschuss der Verbindung der Formel XIX oder einer anorganischen oder organischen Base mit oder ohne Lösungsmittel oder

c) in Gegenwart einer Säure in einem inerten Lösungsmittel bei jeweils Temperaturen von 60° bis 120°C, bevorzugt 80° bis 100°C; oder

B) Thioharnstoff der Formel XX

$$H_2N\text{-}CS\text{-}NH_2 \qquad (XX)$$

mit einem Diketon der Formel III

$$R_3\text{-}CO\text{-}CH_2\text{-}CO\text{-}CH(OR_9)_2 \qquad (III)$$

in Gegenwart einer Säure in einem inerten Lösungsmittel bei Temperaturen von 20°C bis 140°C, bevorzugt 20°C bis 60°C, zu einer Pyrimidinverbindung der Formel XXI

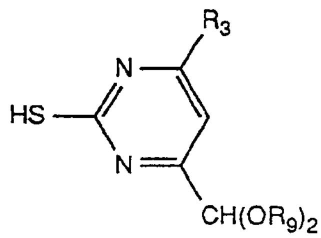

(XXI)

cyclisiert und deren Alkali- oder Erdalkalisalz mit einer Verbindung der Formel XXII

$$ZR_{10} \qquad (XXII),$$

wobei $R_{10}$ $C_1$-$C_8$-Alkyl oder unsubstituiertes oder mit Halogen und/oder $C_1$-$C_4$-Alkyl substituiertes Benzyl und Z Halogen bedeuten, zu einer Pyrimidinverbindung der Formel XXIII

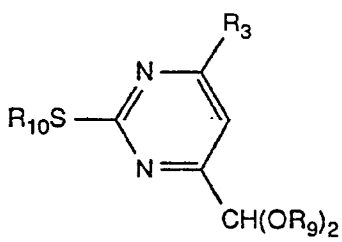

(XXIII)

umsetzt, oder

ein Isothiuroniumsalz der Formel XXIV

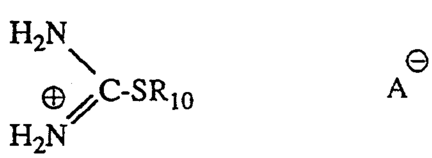

(XXIV)

mit einem Diketon der Formel III, bevorzugt in einem protischen Lösungsmittel, bei Temperaturen von 20°C bis 140°C, bevorzugt 20°C bis 80°C, und ebenfalls zu einer Pyrimidinverbindung der Formel XXIII cyclisiert; und die erhaltene Verbindung der Formel XXIII mit einem Oxydationsmittel, z.B. einer Persäure, zu einer

Pyrimidinverbindung der Formel XXV

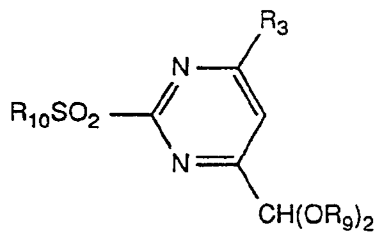

(XXV)

oxydiert und die erhaltene Verbindung der Formel XXV mit einem Formylanilin der Formel XXVI

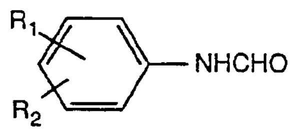

(XXVI)

in einem inerten Lösungsmittel in Gegenwart einer Base als Protonenakzeptor bei Temperaturen zwischen -30°C bis 120°C zu einer Verbindung der Formel XXVII

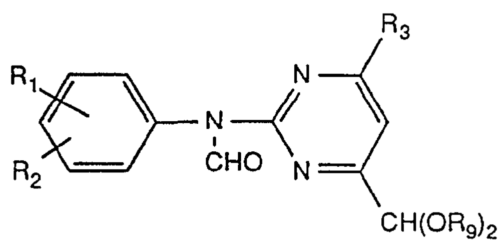

(XXVII)

umsetzt; und

die erhaltene Verbindung der Formel XXVII einer Hydrolyse in Gegenwart einer Base, z.B. Alkalihydroxid, oder einer Säure, z.B. Halogenwasserstoffsäure oder Schwefelsäure, in Wasser oder wässrigen Lösungsmittelgemischen, wie wässerigen Alkoholen oder Dimethylformamid, bei Temperaturen von 10°C bis 110°C, bevorzugt 30°C bis 60°C, unterwirft, wobei in den Formeln XVII bis XXVII die Substituenten $R_1$ bis $R_3$ die unter Formel I angegebenen Bedeutungen besitzen und $A^{\ominus}$ ein Säureanion darstellt.

Falls die Hydrolyse der Verbindungen der Formel XXVII in Gegenwart höherer Säurekonzentrationen und höherer Temperaturen bei längeren Reaktionszeiten durchgeführt wird, wird anstelle des Pyrimidincarbaldehydacetals der Formel IV der Pyrimidincarbaldehyd der Formel V erhalten.

In den beschriebenen Verfahren kommen bei den Verbindungen der Formeln IIa für das Säureanion $A^{\ominus}$ beispielsweise folgende Salzreste in Betracht: Carbonat, Hydrogencarbonat, Nitrat, Halogenid, Sulfat oder Hydrogensulfat. Bei den Verbindungen der Formel XXIV werden als Säureanion $A^{\ominus}$ Sulfat, Hydrogensulfat oder Halogenid bevorzugt.

Unter Halogenid sind jeweils Fluorid, Chlorid, Bromid oder Jodid, bevorzugt Bromid oder Chlorid, zu verstehen.

Als Säuren finden vornehmlich anorganische Säuren, wie z.B. Halogenwasserstoffsäuren, beispielsweise Fluorwasserstoffsäure, Chlorwasserstoffsäure oder Bromwasserstoffsäure, sowie Schwefelsäure, Phosphorsäure oder Salpetersäure Verwendung; jedoch können auch geeignete organische Säuren wie Essigsäure, Toluolsulfonsäure u.a. verwendet werden.

Als Protonenakzeptoren dienen z.B. anorganische oder organische Basen, wie beispielsweise Alkali- oder Erdalkaliverbindungen, z.B. die Hydroxide, Oxide oder Carbonate von Lithium, Natrium, Kalium, Magnesium, Calcium, Strontium und Barium oder auch Hydride wie z.B. Natriumhydrid. Als organische Basen seien beispielsweise tertiäre Amine wie Triethylamin, Triethylendiamin, Pyridin genannt.

In den vorstehend beschriebenen Verfahren können in Anpassung an die jeweiligen Reaktionsbedingungen beispielsweise folgende Lösungsmittel neben den z.T. genannten verwendet werden:

Halogenkohlenwasserstoffe, z.B. Chlorkohlenwasserstoffe, wie Tetrachlorethan, Methylenchlorid, Chloroform, Tetrachlorkohlenstoff, Chlorbenzol, Chlortoluol; Ether, wie Ethylpropylether, Methyl-tert.-butylether, Din-butylether, Di-isobutylether, Diisoamylether, Diisopropylether, Diethylether, Ethylenglykoldimethylether, Tetrahydrofuran, Dioxan; Nitrokohlenwasserstoffe wie Nitrobenzol; Nitrile wie Acetonitril, Butyronitril, Isobutyronitril; aliphatische oder cycloaliphatische Kohlenwasserstoffe, wie Heptan, Hexan, Benzinfraktionen innerhalb eines Siedepunktintervalles von 70°C bis 190°C, Cyclohexan, Petrolether, Ligroin; Ester wie Ethylacetat; Amide, wie Formamid, Dimethylformamid; Ketone, wie Aceton, Methylethylketon; Alkohole, insbesondere niedere aliphatische Alkohole, wie z.B. Methanol, Ethanol, n-Propanol, iso-Propanol sowie die Isomeren der Butanole; gegebenenfalls auch Wasser. Auch Gemische der genannten Lösungs- und Verdünnungsmittel kommen in Betracht.

Analoge Synthesemethoden zu den vorstehend beschriebenen Herstellungsverfahren sind in der Literatur publiziert. Als Hinweise seien genannt: A.Keutzberger und J.Gillessen, J.Heterocyclic Chem.22, 101 (1985); O. Stark, Ber.Dtsch.Chem.Ges.42, 699 (1909); J. Hale, J.Am.Chem.Soc.36, 104 (1914); G.M. Kosolapoff, J.Org.Chem.26, 1895 (1961). St. Angerstein, Ber.Dtsch.Chem.Ges.34, 3956 ( 1901 ); M.P.V. Boarland and J.F.W. McOmie, J.Chem.Soc. 1951, 1218; T.Matsukawa und K. Shirakuwa, J.Pharm.Soc.Japan 71, 933 (1951 ); Chem.Abstr.46, 4549 ( 1952).

Die beschriebenen Herstellungsverfahren einschliesslich aller Teilschritte sind ein Bestandteil der vorliegenden Erfindung.

Folgende Verbindungen, die bei der Herstellung der Verbindungen der Formel I als Zwischenprodukte verwendet werden, sind neu und stellen einen Teil der vorliegenden Erfindung dar:

Verbindungen der Formel V'

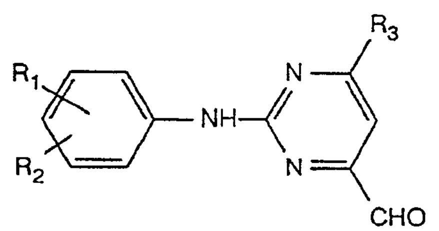

(V'),

in welcher bedeuten:

$R_1$ und $R_2$ unabhängig voneinander Wasserstoff, Halogen, $C_1$-$C_3$-Alkyl, $C_1$-$C_2$-Halogenalkyl, $C_1$-$C_3$-Alkoxy oder $C_1$-$C_3$-Halogenalkoxy:

$R_3$ $C_1$-$C_4$-Alkyl oder durch Halogen oder Hydroxy substituiertes $C_1$-$C_2$-Alkyl.

Substituierte N-PyrimidinylanilinDerivate sind bereits bekannt. So sind in der publizierten europäischen Patentanmeldungen Nr. 0 224 339 und Nr. 270 111 sowie der DD-Patentschrift 151 404 Verbindungen, die eine N-2-Pyrimidinylstruktur aufweisen, als wirksam gegen pflanzenschädigende Fungi beschrieben. Die bekannten Verbindungen konnten jedoch bisher die in der Praxis an sie gestellten Forderungen nicht in vollem Masse befriedigen. Die erfindungsgemässen Verbindungen der Formel I unterscheiden sich in charakteristischer Weise von den bekannten Verbindungen durch die Einführung spezifischer Substituenten und deren Kombination in die Anilinopyrimidin-Struktur, wodurch bei den neuen Verbindungen der Formel I eine unerwartet hohe fungizide Wirksamkeit erreicht wird.

Ueberraschenderweise wurde nun gefunden, dass Verbindungen der Formel I ein für praktische Bedürfnisse sehr günstiges biozides Spektrum zur Bekämpfung von Insekten und phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten

Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem übefluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen für die direkte Pflanzenbehandlung oder die Bodenbehandlung liegen im allgemeinen bei 50 g bis 5 kg Aktivsubstanz (AS) je ha, bevorzugt 100 g bis 2 kg AS/ha, insbesondere bei 200 g bis 600 g AS/ha. Die Mengen an Wirkstoff für gebeiztes und ausgesätes Saatgut liegen deutlich niedriger bei 0,1 g bis 500 g AS/ha, bevorzugt bei 0,5 g bis 100 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstcffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein,

Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4- 14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykoletherguppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,9 bis 5 Gew.-%, eines festen oder flüssigen Zusatzstoffes und 0 bis 25 Gew.-%, insbesondere 0,1 bis 25 Gew.-%, eines Tensides. Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

1. Herstellungsbeispiele

Beispiel 1.1: Herstellung von 2-Anilino-4-formyl-0-methyloxim-6-methyl-pyrimidin

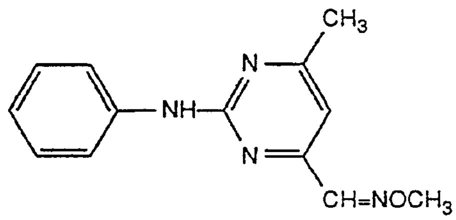

(Verb. Nr. 1.4)

21,3 g (0,1 Mol) 2-Anilino-4-formyl-6-methyl-pyrimidin in 200 ml Ethanol werden unter Rühren bei Raumtemperatur innerhalb einer halben Stunde portionenwiese zunächst mit 9,2 g (0,11 Mol) O-Methylhydroxylaminhydrochlorid, dann mit 12 g (0,11 Mol) $Na_2CO_3$ versetzt und anschliessend unter Rühren 6 Stunden unter Rückfluss erhitzt. Nach dem Abkühlen auf Raumtemperatur wird das Lösungsmittel verdampft und der Rückstand in 200 ml Wasser gelöst. Nach dreimaliger Extraktion mit je 250 ml Diethylether werden die vereinigten Etherextrakte mit 40 ml Wasser gewaschen, über Natriumsulfat getrocknet, filtriert und der Diethylether verdampft. Der zurückbleibende gelb gefärbte Festkörper wird zur Reinigung aus Ethanol umkristallisiert. Die gelblichen Kristalle schmelzen bei 75-77°C.

Beispiel 1.2: Herstellung von 2-Anilino-4-formyl-(0-methoxycarbonyl-oxim)-6-cyclopropyl-pyrimidin

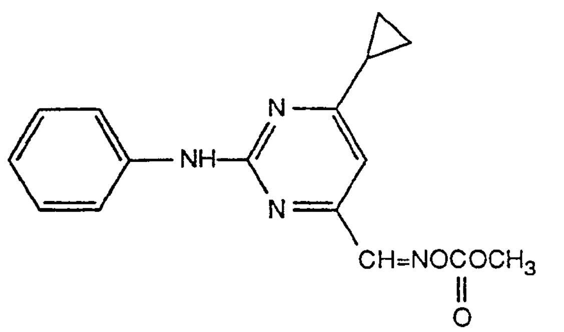

(Verb. Nr. 1.10)

Zu 7,6 g (30 mMol) 2-Anilino-4-formyloxim-6-cyclopropyl-pyrimidin und 3,8 g (38 mMol) Triethylamin in 200 ml abs. Toluol werden bei Raumtemperatur 3,8 g (40 mMol) Chlorameisensäuremethylester in 50 ml abs. Toluol innerhalb einer halben Stunde zugetropft und noch 2 Stunden bei Raumtemperatur gerührt. Nach dreimaligem Waschen mit je 50 ml Wasser wird die Reaktionslösung über Natriumsulfat getrocknet, filtriert und das Lösungsmittel verdampft. Der zurückbleibende gelbe Oel wird durch Behandeln mit Diisopropylether zur Kristallisation gebracht und der gelbe Festkörper durch Umkristallisation aus Methanol gereinigt. Die gelblichen Kristalle schmelzen bei 114-115°C.

Beispiel 1.3: Herstellung von 2-Anilino-4-formyl-(2-ethoxyethylimin)-6-cyclopropylpyrimidin

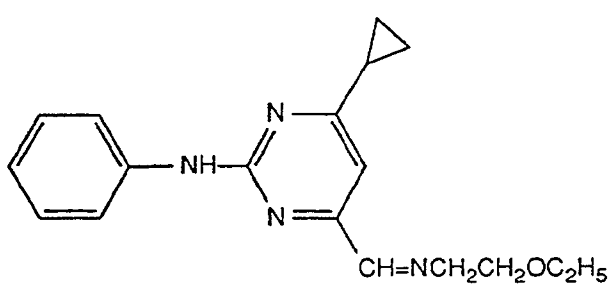

(Verb. Nr. 2.1)

20 g (80 mMol) 2-Anilino-4-formyl-6-cyclopropyl-pyrimidin, 8,4 g (94 mMol) Ethoxyethylamin und 200 mg katalytisch wirkende p-Toluolsulfonsäure in 250 ml Cyclohexan werden 20 Stunden unter Rückfluss erhitzt, wobei sich bildendes Wasser durch einen Wasserabscheider abgetrennt wird. Nach dem Abkühlen auf Raumtemperatur wird das Lösungsmittel abgedampft, der ölige Rückstand in 300 ml Diethylether gelöst, zweimal mit je 20 ml Wasser gewaschen, über Natriumsulfat getrocknet, filtriert und das Lösungsmittel verdampft und der ölige Rückstand durch Anreiben mit Petrolether (40-60°C) zur Kristallisation gebracht. Nach dem Umkristallisieren aus Methanol schmelzen die gelblichen Kristalle bei 88-89°C.

Beispiel 1.4: Herstellung von 2-Anilino-4-formyl-dimethylhydrazon-6-cyclopropylpyrimidin

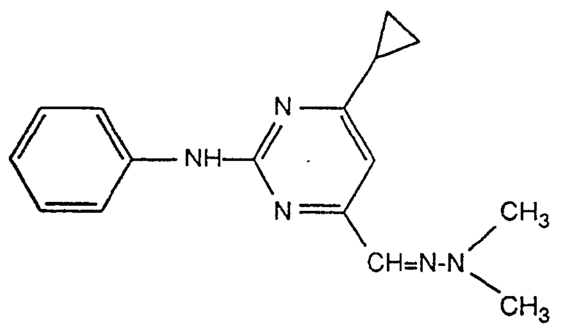

(Verb. Nr. 2.23)

20 g (0,08 Mol) 2-Anilino-4-formyl-6-cyclopropyl-pyrimidin, 14,4 g (0,24 Mol) Dimethylhydrazin und 1 ml Eisessig in 50 ml Ethanol werden 16 Stunden unter Rückfluss erhitzt. Nach dem Abdampfen des Lösungsmittels wird der gelbe Rückstand aus Methanol umkristallisiert. Die gelben Kristalle schmelzen bei 109-110°C.

Beispiel 1.5: Herstellung von 2-Anilino-4-methylamino-N-isopropyl-6-cyclopropylpyrimidin

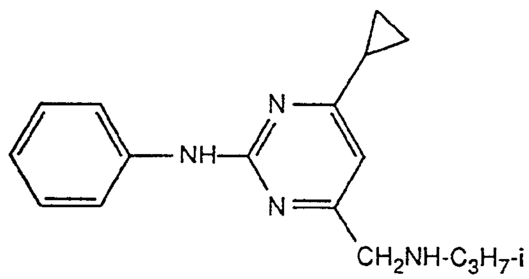

(Verb. Nr. 3.5)

20 g (84 mMol) 2-Anilino-4-formyl-6-cyclopropyl-pyrimidin und 6 g (0,1 Mol) Isopropylamin in 300 ml Ethylacetat werden innerhalb von 3 Stunden unter Normaldruck und unter Verwendung von 1,5 %igem Palladium/Kohle-Katalysator bei Raumtemperatur hydriert. Nach beendeter Wasserstoffaufnahme wird vom Katalysator abfiltriert, das Lösungsmittel abgedampft und der schwach gelbgefärbte Festkörper aus Toluol/Diisopropylether umkristallisiert. Die gelben Kristalle schmelzen bei 73-74°C.

Beispiel 1.6: Herstellung von 2-Anilino-4-(methylamino-N-cyclopropyl)-6-methylpyrimidin

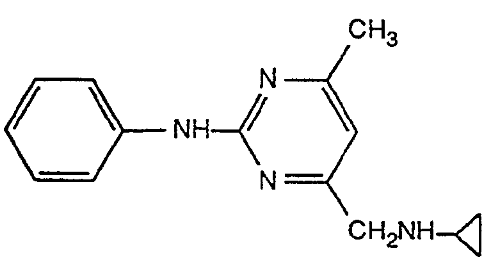

(Verb. Nr. 3.10)

5 g (33 mMol) 2-Anilino-4-formyl-(cyclopropylimin)-6-methyl-pyrimidin in 100 ml Ethylacetat werden innerhalb von 1,5 Stunden unter Normaldruck mittels 0,5 %igem Palladium/Kohle-Katalysator bei Raumtemperatur

hydriert. Nach Aufnahme der berechneten Wasserstoffmenge wird vom Katalysator abfiltriert, das Lösungsmittel verdampft und der ölige Rückstand durch Zugabe von wenig Diisopropylether zur Kristallisation gebracht. Nach dem Umkristallisieren aus Diisopropylether schmelzen die gelblichen Kristalle bei 78-79°C.

Beispiel 1.7: Herstellung von 2-Anilino-4-methylamino-6-methyl-pyrimidin

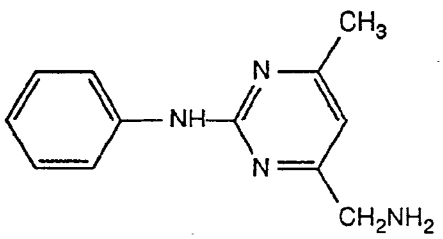

(Verb. Nr. 3.3)

10 g (44 mMol) 2-Anilino-4-formyloxim-6-methyl-pyrimidin in 300 ml Ethylacetat und 100 ml Tetrahydrofuran werden innerhalb von 15 Stunden bei einem Druck von $10^6$ Pa mittels 3 g Raney-Nickel-Katalysator bei ca. 30°C hydriert. Nach beendeter Wasserstoffaufnahme wird vom Katalysator abfiltriert, das Lösungsmittel verdampft und der zunächst ölige Rückstand mit wenig Ethylacetat/Diisopropylether zur Kristallisation gebracht, filtriert und der Rückstand aus Methanol umkristallisiert. Die hellgelben Kristalle schmelzen bei 113-115°C.

Herstellung der Zwischenprodukte

Beispiel 1.8: Herstellung von 2-Anilino-4-formyldiethylacetal-6-methyl-pyrimidin

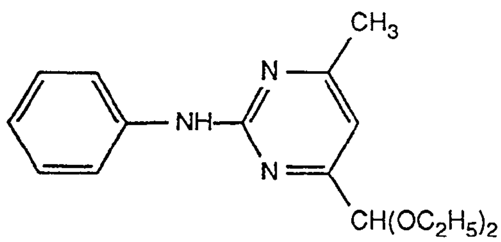

11,8 g (60 mMol) Phenylguanidin-hydrogencarbonat und 12 g (64 mMol) 1-Methyl-3-formyldiethylacetat- 1,3-propandion in 80 ml Ethanol werden unter Rühren 4 Stunden unter Rückfluss erhitzt, wobei die Kohlendioxidentwicklung mit fortschreitender Reaktionsdauer nachlässt. Nach dem Abkühlen wird das Ethanol weitgehend verdampft, der Rückstand in 300 ml Diethylether aufgenommen, dreimal mit je 30 ml Wasser gewaschen, über Natriumsulfat getrocknet, filtriert und der Diethylether verdampft. Das zurückbleibende dunkelbraune Oel wird in 350 ml Petrolether (40-60°C) in der Wärme gelöst in Gegenwart von Aktivkohle 10 Minuten unter Rückfluss erhitzt, nach dem Abkühlen auf ca. 40°C über Hyflo filtriert und auf ein Volumen von etwa 30 ml eingeengt. Die ausgefallenen gelblichen Kristalle werden abfiltriert und schmelzen bei 63-65°C.

Beispiel 1.9: Herstellung von 2-Anilino-4-formyl-6-methyl-pyrimidin

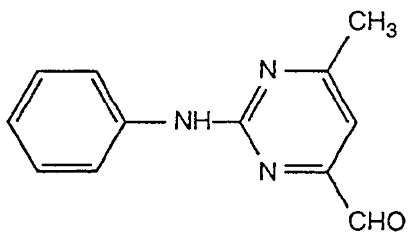

3 g (10,4 mMol) 2-Anilino-4-formyldiethylacetal-6-methyl-pyrimidin und 1 g konz. Salzsäure in 30 ml Wasser werden 24 Stunden bei 45°C gerührt, auf 15°C gekühlt und mit Natriumhydrogencarbonatlösung der pH-Wert

auf 8 eingestellt. Das hellbraune Pulver wird abfiltriert, mit Wasser gewaschen und aus 70 ml Acetonitril umkristallisiert. Die gelben Kristalle schmelzen bei 130-131°C.

<u>Tabelle 1:</u> Verbindungen der Formel

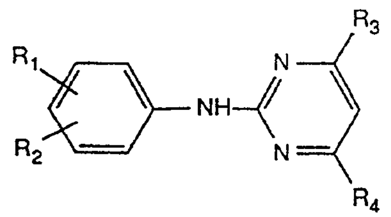

(Ph = Phenyl)

| Verb. Nr. | $R_1$ | $R_2$ | $R_3$ | $R_4$ | Physikalische Konstante |
|---|---|---|---|---|---|
| 1.1 | H | H | $-CH_3$ | -CH=NOH | Smp. 240-241°C |
| 1.2 | 3-F | H | 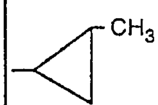 | -CH=NOH | |
| 1.3 | H | H | $-CH_3$ | $-CH=NOCH_2CH_2Cl$ | |
| 1.4 | H | H | $-CH_3$ | $-CH=NOCH_3$ | Smp. 75-77°C |
| 1.5 | $4-CH_3$ | H | $-CH_3$ | $-CH=NOCH_3$ | |
| 1.6 | H | H | Cyclopropyl | $-CH=NOCH_3$ | Smp. 103-104°C |
| 1.7 | H | H | Cyclopropyl | -CH=NOH | Smp. 175-185°C |
| 1.8 | $3-CF_3$ | H | $-CH_3$ | $-CH=NOCH_3$ | |
| 1.9 | 3-F | H | 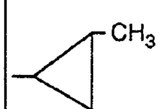 | $-CH=NOCH_3$ | |
| 1.10 | H | H | Cyclopropyl | $-CH=NOCOCH_3$<br>II<br>O | Smp. 114-115°C |
| 1.11 | 3-F | H | $-CH_2Cl$ | $-CH=NOCH_3$ | |
| 1.12 | H | H | $-CH_3$ | $-CH_2=NOCH_2CH_2OH$ | |
| 1.13 | H | H | $-CH_2Cl$ | -CH=NOH | |
| 1.14 | H | H | $-C_2H_5$ | -CH=NOH | |

Fortsetzung: Tabelle 1

| Verb. Nr. | $R_1$ | $R_2$ | $R_3$ | $R_4$ | Physikalische Konstante |
|---|---|---|---|---|---|
| 1.15 | 3-F | 4-Cl | Cyclopropyl | $-CH=NOCH_3$ | |
| 1.16 | H | H | $-CH_3$ | $-CH=NOC_2H_5$ | |
| 1.17 | 3-F | H | $-CH_2Cl$ | -CH=NOH | |
| 1.18 | $4-OCH_3$ | H | $-CH_3$ | $-CH=NOCH_3$ | |
| 1.19 | H | H | Cyclopropyl | $-CH=NOCH_2$ -Ph | Smp. 107-108°C |
| 1.20 | H | H | $-CH_3$ | $-CH=NOCH_2COOCH_3$ | |
| 1.21 | 3-F | H | 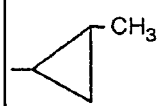 | $-CH_2=NOCH_2C\equiv CH$ | |
| 1.22 | H | H | $-CH_3$ | $-CH=NO-CH_2$ -Ph | |
| 1.23 | 4-F | H | $-CH_3$ | -CH=NOH | Smp. 194-196°C |
| 1.24 | H | H | Cyclopropyl | $-CH=NOCNHCH_3$<br>‖<br>O | Smp. 135-136°C (Zers.) |
| 1.25 | 3-F | 4-Cl | Cyclopropyl | -CH=NOH | |
| 1.26 | H | H | $-CH_3$ | $-CH=NOCH_2-CH=CH_2$ | |
| 1.27 | 3-F | H | $-CH_2F$ | $-CH=NOCH_3$ | |
| 1.28 | $3-OCHF_2$ | H | $-CH_3$ | $-CH=NOCH_3$ | |
| 1.29 | H | H | $-C_3H_7$-n | -CH=NOH | |
| 1.30 | H | H | $-CH_2Cl$ | $-CH=NOCH_3$ | |
| 1.31 | 4-F | H | $-CH_3$ | $-CH=NOCH_3$ | Smp.109-111°C |
| 1.32 | 3-F | H | Cyclopropyl | $-CH=NOCH_2-CH=CH_2$ | |
| 1.33 | 3-F | 4-Cl | $-CH_3$ | $-CH=NOCH_3$ | |
| 1.34 | H | H | $-CH_3$ | $-CH=NOCH_2C\equiv CH$ | |
| 1.35 | H | H | Cyclopropyl | $-CH=NOC_2H_5$ | Smp. 79-81°C |
| 1.36 | H | H | $-C_3H_7$-n | $-CH=NOCH_3$ | |
| 1.37 | 3-F | H | $-CH_2F$ | -CH=NOH | |

Fortsetzung: Tabelle 1

| Verb. Nr. | $R_1$ | $R_2$ | $R_3$ | $R_4$ | Physikalische Konstante |
|---|---|---|---|---|---|
| 1.38 | H | H | $-CH_2OH$ | -CH=NOH | |
| 1.39 | H | H | Cyclopropyl | $-CH=NOC_2CH_2Cl$ | |
| 1.40 | 3-F | 4-Cl | $-CH_3$ | -CH=NOH | |
| 1.41 | 3-F | H | Cyclopropyl | $-CH=NOCH_3$ | |
| 1.42 | H | H | $-CH_3$ | $-CH=NOCCH_3$<br>‖<br>O | |
| 1.43 | 4-F | H | $-CH_3$ | $-CH=NOCH_2-CH=CH_2$ | |
| 1.44 | H | H | $-CH_2OH$ | $-CH=NOCH_3$ | |
| 1.45 | H | H | $-C_2H_5$ | $-CH=NOCH_3$ | |
| 1.46 | H | H | Cyclopropyl | $-CH=NOCH_2COOCH_3$ | |
| 1.47 | H | H | 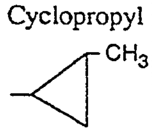 | $-CH=NOCCH_3$<br>‖<br>O | |
| 1.48 | 3-F | H | Cyclopropyl | -CH=NOH | |
| 1.49 | 3-F | 4-F | Cyclopropyl | -CH=NOH | |
| 1.50 | H | H | $-CH_3$ | $-CH=NOCCH_2Cl$<br>‖<br>O | |
| 1.51 | 4-F | H | $-CH_3$ | $-CH=NOCH_2-C\equiv CH$ | |
| 1.52 | H | H | 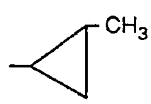 | $-CH=NOC_2H_5$ | |
| 1.53 | H | H | $-C_2H_5$ | $-CH=NOC_2H_5$ | |
| 1.54 | H | H | Cyclopropyl | $-CH=NOCCH_3$<br>‖<br>O | |
| 1.55 | H | H | Cyclobutyl | -CH=NOH | |
| 1.56 | 3-F | H | $-C_2H_5$ | $-CH=NOCH_3$ | |

Fortsetzung: Tabelle 1

| Verb. Nr. | $R_1$ | $R_2$ | $R_3$ | $R_4$ | Physikalische Konstante |
|---|---|---|---|---|---|
| 1.57 | H | H | $-CH_3$ | $-CH=NO\overset{O}{\overset{\|}{C}}CH_2OCH_3$ | |
| 1.58 | 3-F | 4-F | Cyclopropyl | $-CH=NOH$ | |
| 1.59 | 4-F | H | $-CH_3$ | $-CH=NO\overset{O}{\overset{\|}{C}}CH_3$ | |
| 1.60 | H | H | Cyclopropyl | $-CH=NO\overset{O}{\overset{\|}{C}}CH_2Cl$ | |
| 1.61 | H | H | $-C_2H_5$ | $-CH=NO\overset{O}{\overset{\|}{C}}CH_3$ | |
| 1.62 | H | H | Cyclohexyl | $-CH=NOCH_3$ | |
| 1.63 | H | H | $-C_2H_5$ | $-CH=NOCH_2-C\equiv CH$ | |
| 1.64 | 3-F | H | $-C_2H_5$ | $-CH=NOH$ | |
| 1.65 | H | H | Cyclopropyl | $-CH=NO\overset{O}{\overset{\|}{C}}CH_2OCH_3$ | |
| 1.66 | 4-F | H | Cyclopropyl | $-CH=NOH$ | |
| 1.67 | H | H | $-CH_3$ | 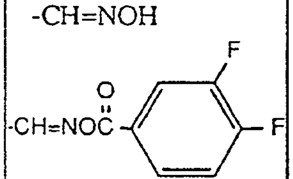 | |
| 1.68 | 3-F | 4-F | $-CH_3$ | $-CH=NOCH_3$ | |
| 1.69 | H | H | $-C_2H_5$ | $-CH=NO\overset{O}{\overset{\|}{C}}CH_2Cl$ | |
| 1.70 | H | H | $-C_2H_5$ | $-CH=NO\overset{O}{\overset{\|}{C}}NCH_3$ | |
| 1.71 | 3-F | H | $-CH_3$ | $-CH=NO\overset{O}{\overset{\|}{C}}CH_3$ | |

Fortsetzung: Tabelle 1

| Verb. Nr. | $R_1$ | $R_2$ | $R_3$ | $R_4$ | Physikalische Konstante |
|---|---|---|---|---|---|
| 1.72 | H | H | $-CH_2F$ | $-CH=NOCH_2-C\equiv CH$ | |
| 1.73 | 4-F | H | Cyclopropyl | $-CH=NOCH_3$ | |
| 1.74 | H | H | $-CH_3$ | $-CH=NO\underset{\underset{O}{\|}}{C}NHCH_3$ | |
| 1.75 | 3-F | 4-F | $-CH_3$ | $-CH=NOH$ | |
| 1.76 | H | H | $-CH_3$ | 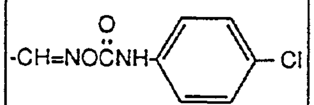 | |
| 1.77 | 3-F | H | $-CH_3$ | $-CH=NO\underset{\underset{O}{\|}}{C}NHCH_3$ | Smp. 171-172°C |
| 1.78 | H | H | $-CH_2F$ | $-CH=NO\underset{\underset{O}{\|}}{C}NH-CH_3$ | |
| 1.79 | H | H | $-CH_2F$ | $-CH=NOCH_2-CH=CH_2$ | |
| 1.80 | 4-F | H | Cyclopropyl | $-CH=NOCH_2-CH=CH_2$ | |
| 1.81 | H | H | 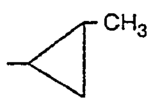 | $-CH=NOH$ | |
| 1.82 | 4-F | H | 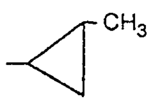 | $-CH=NOCH_3$ | |
| 1.83 | 3-F | H | $-CH_3$ | $-CH=NOCH_2-CH=CH_2$ | |
| 1.84 | H | H | Cyclobutyl | $-CH=NOCH_3$ | |
| 1.85 | H | H | $-CH_3$ | $-CH=NO\underset{\underset{O}{\|}}{C}OCH_3$ | |
| 1.86 | H | H | $-CH_2F$ | $-CH=NOH$ | |
| 1.87 | 4-F | H | Cyclopropyl | $-CH=NOCH_3$ | |

Fortsetzung: Tabelle 1

| Verb. Nr. | $R_1$ | $R_2$ | $R_3$ | $R_4$ | Physikalische Konstante |
|---|---|---|---|---|---|
| 1.88 | H | H | $-CH_2F$ | $-CH=NOCH_3$ | |
| 1.89 | 3-F | H | $-CH_3$ | $-CH=NOCH_3$ | Smp. 81-81,5°C |
| 1.90 | H | H | Cyclohexyl | -CH=NOH | |
| 1.91 | 4-F | H | 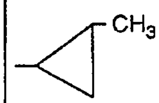 | -CH=NOH | |
| 1.92 | H | H | $-C_2H_5$ | $-CH=NO\overset{\overset{O}{\|}}{C}CH_3$ | |
| 1.93 | 3-F | H | $-CH_3$ | -CH=NOH | > 235°C |
| 1.94 | H | H | 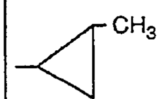 | $-CH=NOCH_3$ | |

Tabelle 2: Verbindungen der Formel

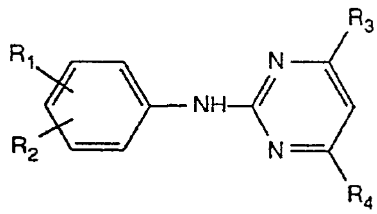

(Ph=Phenyl)

| Verb. Nr. | $R_1$ | $R_2$ | $R_3$ | $R_4$ | Physikalische Konstante |
|---|---|---|---|---|---|
| 2.1 | H | H | Cyclopropyl | $-CH=NCH_2CH_2OC_2H_5$ | Smp. 88-89°C |
| 2.2 | H | H | $-CH_3$ | -CH=N- 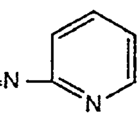 | |
| 2.3 | H | H | $-CH_3$ | -CH=N- 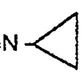 | Smp. 111-112°C |
| 2.4 | H | H | $-CH_3$ | $-CH=N-CH_3$ | |
| 2.5 | H | H | $-C_2H_5$ | $-CH=N-C_2H_5$ | |
| 2.6 | H | H | Cyclopropyl | $-CH=N-C_2H_5$ | |
| 2.7 | H | H | $-CH_3$ | -CH=N- 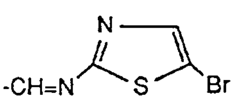 | |
| 2.8 | H | H | $-CH_3$ | -CH=N- 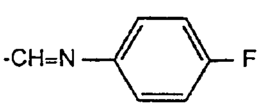 | Smp. 142-143°C |
| 2.9 | H | H | $-CH_3$ | $-CH=N-C_2H_5$ | |
| 2.10 | H | H | Cyclopropyl | $-CH=N-C_3H_7$-n | |
| 2.11 | H | H | $-C_2H_5$ | $-CH=N-C_3H_7$-n | |
| 2.12 | H | H | Cyclopropyl | -CH=N- 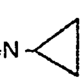 | Smp. 82-83°C |

Fortsetzung: Tabelle 2

| Verb. Nr. | $R_1$ | $R_2$ | $R_3$ | $R_4$ | Physikalische Konstante |
|---|---|---|---|---|---|
| 2.13 | H | H | $-CH_3$ | $-CH=N-CH_2-$ 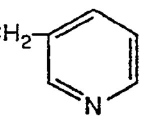 | |
| 2.14 | H | H | Cyclopropyl | $-CH=NCH_2CH_2OCH_3$ | |
| 2.15 | H | H | $-CH_3$ | -CH=N-NH-Ph | |
| 2.16 | H | H | Cyclopropyl | $-CH=N-CH_2CH_2OH$ | |
| 2.17 | H | H | $-CH_3$ | $-CH=N-NH-C-NH_2$<br>‖<br>O | |
| 2.18 | H | H | $-CH_3$ | $-CH=NC_2H_4OC_2H_5$ | Smp. 42-43°C |
| 2.19 | H | H | $-C_2H_5$ | $-CH=NCH_2CH_2OCH_3$ | |
| 2.20 | H | H | $-CH_3$ | $-CH=N-C_3H_7$-n | |
| 2.21 | H | H | Cyclopropyl | $-CH=N-CH_2-CH-CH_2$<br>OH OH | |
| 2.22 | H | H | 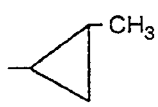 | $-CH=NCH_2$-Ph | |
| 2.23 | H | H | Cyclopropyl | 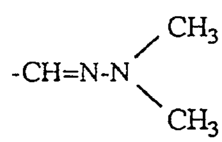 | Smp. 109-110°C |
| 2.24 | H | H | 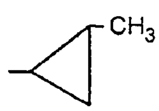 | $-CH=NCH_2-$ 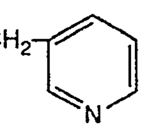 | |
| 2.25 | H | H | Cyclopropyl | 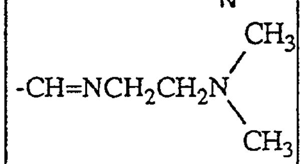 | |
| 2.26 | H | H | $-CH_3$ | $-CH=N_2H_4OCH_3$ | |
| 2.27 | H | H | $-CH_3$ | $-CH=N-NH-C-NH_2$<br>‖<br>S | |
| 2.28 | H | H | $-C_2H_5$ | $-CH=NCH_2CH_2OH$ | |

Fortsetzung: Tabelle 2

| Verb. Nr. | $R_1$ | $R_2$ | $R_3$ | $R_4$ | Physikalische Konstante |
|---|---|---|---|---|---|
| 2.29 | H | H | $-CH_3$ | $-CH{=}NCH_2CH_2OH$ | |
| 2.30 | H | H | 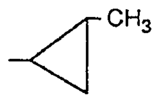 | 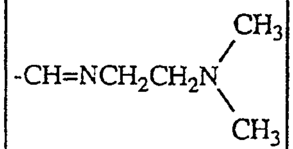 | |
| 2.31 | H | H | $-CH_3$ | 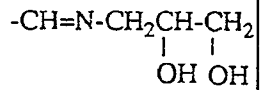 | |
| 2.32 | H | H | Cyclopropyl | 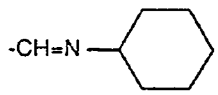 | |
| 2.33 | H | H | $-CH_3$ | 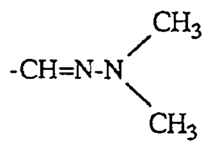 | Smp. 161-161,5°C |
| 2.34 | H | H | 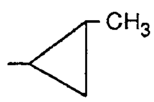 | $-CH{=}N-CH_2CH_2CN$ | |
| 2.35 | H | H | $-C_2H_5$ | 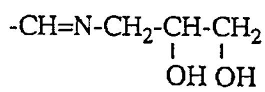 | |
| 2.36 | H | H | $-CH_3$ | $-CH{=}NCH_2-CH{=}CH_2$ | |
| 2.37 | H | H | Cyclopropyl | $-CH{=}NCH_2-Ph$ | |
| 2.38 | 3-F | H | $-CH_3$ | $-CH{=}NCH_2CH_2OCH_3$ | |
| 2.39 | H | H | 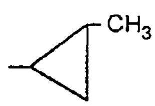 | $-CH{=}NCH_2CH_2OH$ | |
| 2.40 | H | H | $-CH_3$ | $-CH{=}NCH_2CH_2CN$ | |
| 2.41 | H | H | Cycloproyl | $-CH{=}N-CH_2CH{=}CH_2$ | |
| 2.42 | H | H | $-C_2H_5$ | 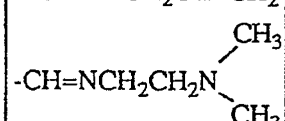 | |

Fortsetzung: Tabelle 2

| Verb. Nr. | $R_1$ | $R_2$ | $R_3$ | $R_4$ | Physikalische Konstante |
|---|---|---|---|---|---|
| 2.43 | H | H | 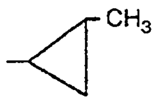 | $-CH=N-CH_2CH_2OCH_3$ | |
| 2.44 | H | H | $-CH_3$ | $CH_3$<br>/<br>$-CH=NCH_2CH_2N$<br>\\<br>$CH_3$ | |
| 2.45 | H | H | Cyclopropyl | -CH=N-NH-Ph | |
| 2.46 | 3-F | H | $-CH_3$ | $CH_3$<br>/<br>$-CH=NCH_2CH_2N$<br>\\<br>$CH_3$ | |
| 2.47 | H | H | $-CH_3$ | -CH=N 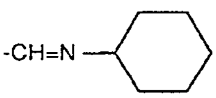 | |
| 2.48 | H | H | 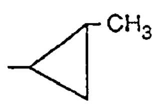 | $-CH=N-C_3H_7-n$ | |
| 2.49 | H | H | Cyclopropyl | $-CH=N-NH-C-NH_2$<br>‖<br>O | |
| 2.50 | 3-F | 4-F | $-CH_3$ | $-CH=N-C_2H_5$ | |
| 2.51 | H | H | $-CH_3$ | $-CH=N-CH_2-Ph$ | |
| 2.52 | H | H | 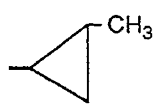 | $-CH=N-C_3H_7-i$ | |
| 2.53 | H | H | $-C_2H_5$ | $-CH=N-CH_2-Ph$ | |
| 2.54 | 3-F | H | $-CH_3$ | -CH=N 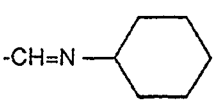 | |
| 2.55 | H | H | $-CH_3$ | -CH=N 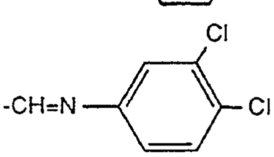 | |

Fortsetzung: Tabelle 2

| Verb. Nr. | $R_1$ | $R_2$ | $R_3$ | $R_4$ | Physikalische Konstante |
|---|---|---|---|---|---|
| 2.56 | 3-F | H | Cyclopropyl | $-CH=N-C_3H_7$-n | |
| 2.57 | H | H | Cyclopropyl | $-CH=N-NH-C(=S)-NH_2$ | |
| 2.58 | 4-F | H | $-CH_3$ | $-CH=N-C_2H_5$ | |
| 2.59 | H | H | $-CH_3$ | $-CH=N-CH(CH_3)-COOCH_3$ | |
| 2.60 | H | H | 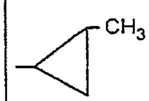 | $-CH=N-C_2H_5$ | |
| 2.61 | H | H | $-CH_3$ | $-CH=N-CH_2-C\equiv CH$ | |
| 2.62 | 4-F | H | Cyclopropyl | $-CH=N-CH(CH_3)-C_2H_5$ | |

Tabelle 3: Verbindungen der Formel

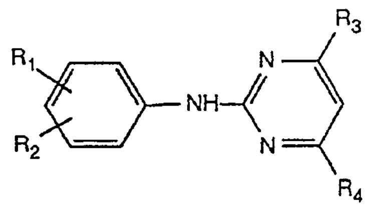

(Ph=Phenyl)

| Verb. Nr. | $R_1$ | $R_2$ | $R_3$ | $R_4$ | Physikalische Konstante |
|---|---|---|---|---|---|
| 3.1 | H | H | $-CH_3$ | $CH_3$<br>$-CH_2NH-CH$<br>$CH_3$ | Smp. 70-72°C |
| 3.2 | H | H | Cyclopropyl | $CH_3$<br>$-CH_2NHCH_2CH$<br>$CH_3$ | Oel; $n_D^{20}$: 1.5958 |
| 3.3 | H | H | $-CH_3$ | $-CH_2NH_2$ | Smp. 113-115°C |
| 3.4 | H | H | $-CH_3$ | $CH_3$<br>$-CH_2NH-CHCOOCH_3$ | |
| 3.5 | H | H | Cyclopropyl | $CH_3$<br>$-CH_2NH-CH$<br>$CH_3$ | Smp. 73-74°C |
| 3.6 | H | H | $-CH_3$ | $-CH_2NH-CH_2-Ph$ | |
| 3.7 | H | H | $-CH_3$ | $CH_3$<br>$-CH_2NHC_2H_4N$<br>$CH_3$ | |

Fortsetzung: Tabelle 3

| Verb. Nr. | $R_1$ | $R_2$ | $R_3$ | $R_4$ | Physikalische Konstante |
|---|---|---|---|---|---|
| 3.8 | H | H | $-CH_3$ | 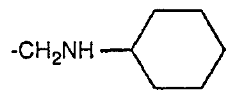 | |
| 3.9 | H | H | $-CH_3$ | $-CH_2NHCH_2CH_2CN$ | |
| 3.10 | H | H | $-CH_3$ | 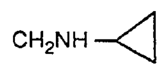 | Smp. 78-79°C |
| 3.11 | H | H | $-CH_3$ | $-CH_2NHC_2H_4OH$ | |
| 3.12 | H | H | Cyclopropyl | $-CH_2NHC_2H_4OH$ | Smp. 109-110°C |
| 3.13 | H | H | $-C_2H_5$ | $-CH_2NHCH_3$ | |
| 3.14 | H | H | $-C_3H_7$ | $-CH_2NHCH_3$ | |
| 3.15 | H | H | Cyclopropyl | $-CH_2NHC_2H_4OC_2H_5$ | Smp. 71-72°C |
| 3.16 | H | H | $-CH_3$ | $-CH_2NHCH_2$-$CH=CH_2$ | |
| 3.17 | 3-F | H | $-CH_3$ | $-CH_2NHCH_3$ | |
| 3.18 | H | H | Cyclopropyl | 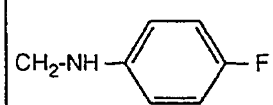 | |
| 3.19 | H | H | $-CH_3$ | $-CH_2NHCH_2CN$ | |
| 3.20 | H | H | $-C_2H_5$ | $-CH_2NHC_2H_5$ | |
| 3.21 | H | H | $-CH_3$ | $-CH_2NH$-Ph | |
| 3.22 | 4-F | H | $-CH_3$ | $-CH_2NHCH_2CH_2OH$ | |
| 3.23 | H | H | Cyclopropyl | $-CH_2NHC_2H_5$ | |
| 3.24 | H | H | $-CH_3$ | $-CH_2NHC_2H_4OC_2H_5$ | |

Fortsetzung: Tabelle 3

| Verb. Nr. | $R_1$ | $R_2$ | $R_3$ | $R_4$ | Physikalische Konstante |
|---|---|---|---|---|---|
| 3.25 | H | H | $-C_2H_5$ | 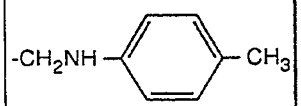 | |
| 3.26 | H | H | Cyclopropyl | $-CH_2NH_2$ | Smp. 142-143°C |
| 3.27 | H | H | $-CH_3$ | 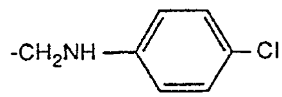 | |
| 3.28 | H | H | $-C_3H_7$-n | $-CH_2NHC_2H_5$ | |
| 3.29 | H | H | Cyclopropyl | 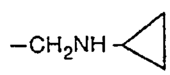 | Smp. 83-84°C |
| 3.30 | H | H | $-C_2H_5$ | $-CH_2NH-C_3H_7$-n | |
| 3.31 | H | H | $-CH_3$ | 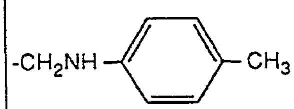 | |
| 3.32 | 3-F | H | $-CH_3$ | $-CH_2NH-C_2H_5$ | |
| 3.33 | H | H | $-C_2H_5$ | $-CH_2CH_2OH$ | |
| 3.34 | H | H | Cyclopropyl | $-CH_2NHCH_3$ | Smp. 92-93°C |
| 3.35 | 4-F | H | $-CH_3$ | $-CH_2NHCH_3$ | |
| 3.36 | H | H | $-CH_3$ | $-CH_2NHC_2H_4OCH_3$ | |
| 3.37 | H | H | $-C_3H_7$-n | $-CH_2NHC_3H_7$-n | |
| 3.38 | H | H | Cyclopropyl | $-CH_2NHC_3H_7$-n | |
| 3.39 | H | H | Cyclopropyl | $-CH_2NHCH_2CH_2Cl$ | |
| 3.40 | H | H | $-CH_3$ | $-CH_2NHCH_2CH_2Cl$ | |
| 3.41 | 3-F | H | $-CH_3$ | $-CH_2NHCH_2CH_2OH$ | |

Fortsetzung: Tabelle 3

| Verb. Nr. | $R_1$ | $R_2$ | $R_3$ | $R_4$ | Physikalische Konstante |
|---|---|---|---|---|---|
| 3.42 | H | H | $-CH_3$ | 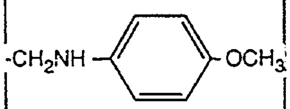 | |
| 3.43 | H | H | Cyclopropyl | $-CH_2NHCH_2CN$ | |
| 3.44 | H | H | $-C_2H_5$ | $-CH_2NHC_2H_4OCH_3$ | |
| 3.45 | 4-F | H | $-CH_3$ | $-CH_2NHC_2H_5$ | |
| 3.46 | H | H | Cyclopropyl | $-CH_2NHC_2H_4N(CH_3)_2$ | Smp. 83-84°C |
| 3.47 | 3-F | H | $-CH_3$ | $-CH_2NHC_2H_4OCH_3$ | |
| 3.48 | H | H | $-CH_3$ | $-CH_2NH-C_3H_7-n$ | |
| 3.49 | H | H | $-C_2H_5$ | $-CH_2NHC_2H_4N(CH_3)_2$ | |
| 3.50 | H | H | $-CH_3$ | 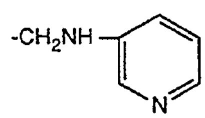 | |
| 3.51 | H | H | Cyclopropyl | $-CH_2NHC_2H_4OCH_3$ | |
| 3.52 | H | H | $-CH_3$ | $-CH_2NHC_4H_9-n$ | |
| 3.53 | H | H | Cyclopropyl | $-CH_2NH-Ph$ | |
| 3.54 | H | H | 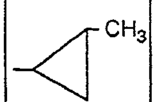 | $-CH_2NHC_2H_4OCH_3$ | |
| 3.55 | H | H | $-C_2H_5$ | $-CH_2NH-CH_2-Ph$ | |
| 3.56 | H | H | Cyclopropyl | $-CH_2NHCH_2CH_2CN$ | |
| 3.57 | H | H | $-CH_3$ | $-CH_2NH-C_2H_5$ | |

Fortsetzung: Tabelle 3

| Verb. Nr. | $R_1$ | $R_2$ | $R_3$ | $R_4$ | Physikalische Konstante |
|---|---|---|---|---|---|
| 3.58 | 3-F | H | $-CH_3$ | 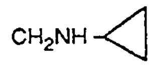 | |
| 3.59 | H | H | Cyclopropyl | $-CH_2NHCH_2$-Ph | |
| 3.60 | H | H | $-CH_3$ | $-CH_2NHCH_3$ | |
| 3.61 | H | H | $-C_3H_5$ | 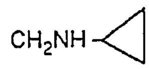 | |
| 3.62 | H | H | Cyclopropyl | 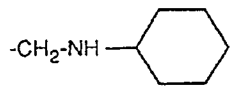 | |
| 3.63 | H | H | 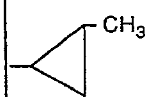 | $-CH_2NHCH_2CH_2OH$ | |
| 3.64 | H | H | Cyclopropyl | 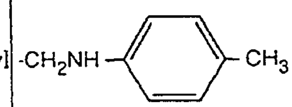 | |
| 3.65 | H | H | 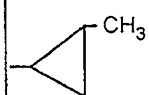 | $-CH_2NH-C_3H_7$-n | |
| 3.66 | H | H | 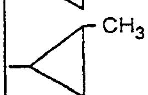 | 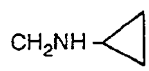 | |
| 3.67 | H | H | 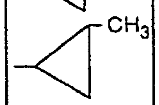 | $-CH_2NHC_2H_5$ | |
| 3.68 | H | H | Cyclopropyl | 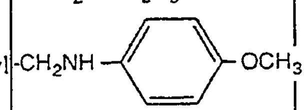 | |
| 3.69 | H | H | 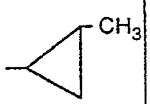 | $-CH_2NHCH_2$-Ph | |
| 3.70 | H | H | 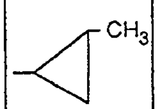 | $-CH_2NHCH_3$ | |

Tabelle 4: Verbindungen der Formel

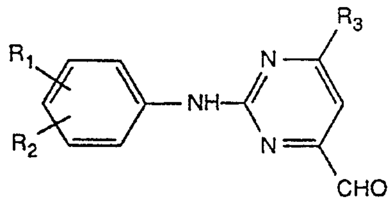

| Verb. Nr. | $R_1$ | $R_2$ | $R_3$ | Physikalische Konstante |
|---|---|---|---|---|
| 4.1 | H | H | $-CH_3$ | Smp. 130-131°C |
| 4.2 | H | H | $-C_2H_5$ | |
| 4.3 | 3-F | H | $-CH_3$ | Smp. 154-155°C |
| 4.4 | 3-F | H | $-CH_2Cl$ | |
| 4.5 | 3-F | 5-F | $-CH_3$ | |
| 4.6 | 4-F | H | $-CH_2F$ | |
| 4.7 | $4-OCH_3$ | H | $-CH_3$ | |
| 4.8 | $3-CF_3$ | H | $-CH_3$ | |
| 4.9 | 3-F | 5-F | $-C_2H_5$ | |
| 4.10 | 4-F | H | $-CH_2Cl$ | |
| 4.11 | 3-Cl | H | $-CH_3$ | |
| 4.12 | $3-CF_3$ | H | $-C_2H_5$ | |
| 4.13 | 3-F | H | $-C_2H_5$ | |
| 4.14 | H | H | $-C_4H_9$-n | |

Fortsetzung: Tabelle 4

| Verb. Nr. | $R_1$ | $R_2$ | $R_3$ | Physikalische Konstante |
|---|---|---|---|---|
| 4.15 | 4-$CH_3$ | H | -$CH_3$ | |
| 4.16 | 4-$OCH_3$ | H | -$C_2H_5$ | |
| 4.17 | 4-$CF_3$ | H | -$CH_3$ | |
| 4.18 | 3-F | H | -$CH_2OH$ | |
| 4.19 | 3-$OCHF_2$ | H | -$CH_3$ | |
| 4.20 | H | H | -$C_3H_7$-n | |
| 4.21 | 4-F | H | -$C_2H_5$ | |
| 4.22 | 3-F | H | -$CH_2CH_2OH$ | |
| 4.23 | 3-F | H | -$CH_2F$ | |
| 4.24 | 3-$CH_3$ | H | -$CH_3$ | |
| 4.25 | 3-F | 5-F | -$CH_2F$ | |
| 4.26 | 3-Cl | H | -$C_2H_5$ | |
| 4.27* | 4-F | H | -$CH_3$ | Smp. 131-133°C |
| 4.28 | H | H | -$C_3H_7$-i | |
| 4.29 | 3-F | H | -$CH_2CH_2Cl$ | |
| 4.30 | 3-$OCHF_2$ | H | -$C_2H_5$ | |
| 4.31 | H | H | -$CH_2Cl$ | |
| 4.32 | 4-$OCHF_2$ | H | -$CH_3$ | |
| 4.33 | H | H | -$CH_2CH_2Cl$ | |
| 4.34 | 3-$CF_3$ | 4-Cl | -$CH_3$ | |
| 4.35 | H | H | -$CH_2F$ | |

* Hydrat (· $H_2O$)

Fortsetzung: Tabelle 4

| Verb. Nr. | $R_1$ | $R_2$ | $R_3$ | Physikalische Konstante |
|---|---|---|---|---|
| 4.36 | 4-Cl | H | $-CH_3$ | |
| 4.37 | $4-OCHF_2$ | H | $-C_2H_5$ | |
| 4.38 | H | H | $-CH_2OH$ | |
| 4.39 | $3-CF_3$ | 4-Cl | $-C_2H_5$ | |
| 4.40 | H | H | $-CH_2CH_2OH$ | |
| 4.41 | 4-Cl | H | $-C_2H_5$ | |

2. Formulierungsbeispiele für flüssige Wirkstoffe der Formel I % = Gewichtsprozent)

| 2.1. Emulsionskonzentrate | a) | b) | c) |
|---|---|---|---|
| Wirkstoff aus den Tabellen | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus der Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abbedampft.

| 2.4. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 2.5. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6. Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentra-

tion hergestellt werden.

| 2.7. Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus den Tabellen | 10 % |
| Na-Ligninsulfonat | 2 % |
| Na-Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Umhüllungs-Granulat | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10. Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Na-Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Venturia inaequalis auf Apfeltrieben Residual-protektive Wirkung

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Verbindungen aus den Tabellen zeigen gegen Venturia gute Wirksamkeit (Befall: weniger als 20 %). So reduzierten z.B. die Verbindungen Nr. 1.4, 2.12 und 3.46 den Venturia-Befall auf 0 bis 10 %. Unbehandelte aber infizierte Kontrollpflanzen weisen dagegen einen Venturia-Befall von 100 % auf.

Beispiel 3.2: Wirkunp gegen Botrytis cinerea an Apfelfrüchten Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgepfropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Verbindungen aus den Tabellen zeigen gegen Botrytis gute Wirksamkeit (Befall: weniger als 20 %). So reduzierten z.B. die Verbindungen Nr. 1.4, 2.1, 2.8, 2.12 und 3.46 den Botrytis-Befall auf 0 bis 10%. Unbehandelte aber infizierte Kontrollpflanzen weisen dagegen einen Botrytis-Befall von 100 % auf.

Beispiel 3.3: Wirkunp gegen Erysiphae graminis auf Gerste Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus den Tabellen zeigen gegen Erysiphae gute Wirksamkeit (Befall: weniger als 20 %). So reduziert z.B. die Verbindung Nr. 1.4 den Erysiphae-Befall auf 0 bis 10 %. Unbehandelte aber infizierte Kontrollpflanzen weisen dagegen einen Erysiphae-Befall von 100 % auf.

Beispiel 3.4: Wirkunp gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen.
Verbindungen aus den Tabellen verhindern den Pilzbefall weitgehend (0 bis 10 % Pilzbefall).

## Patentansprüche

**1.** Verbindungen der Formel I

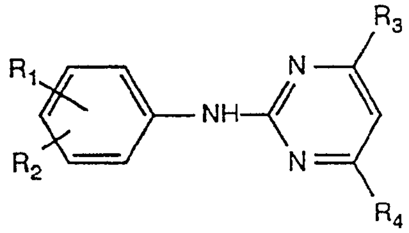

(I)

in welcher bedeuten:
$R_1$ und $R_2$ unabhängig voneinander Wasserstoff, Halogen, $C_1$-$C_3$-Alkyl, $C_1$-$C_2$-Halogenalkyl, $C_1$-$C_3$-Alkoxy oder $C_1$-$C_3$ Halogenalkoxy;
$R_3$ $C_1$-$C_4$-Alkyl, durch Halogen oder Hydroxy substituiertes $C_1$-$C_2$-Alkyl, $C_3$-$C_6$-Cycloalkyl oder durch Methyl oder Halogen bis zu dreifach gleich oder verschieden substituiertes $C_3$-$C_6$-Cycloalkyl;
$R_4$ die Reste $CH{=}NOR_5$, $CH{=}NR_6$ oder $CH_2NHR_6$;
$R_5$ Wasserstoff, $C_1$-$C_8$-Alkyl oder $C_1$-$C_4$-Alkyl, das durch Halogen, Hydroxy, Cyano, $C_1$-$C_4$-Alkoxy, $COOC_1$-$C_3$-Alkyl oder durch Phenyl substituiert ist, wobei das Phenyl durch Halogen, $C_1$-$C_3$-Alkyl, $C_1$-$C_3$-Alkoxy oder Nitro substituiert sein kann;
$R_5$ ferner $C_3$-$C_6$-Alkenyl oder durch Halogen substituiertes $C_3$-$C_6$-Alkenyl; $C_3$-$C_6$-Alkinyl oder durch Halogen substituiertes $C_3$-$C_6$-Alkinyl, oder einen Acylrest COR', worin R' $C_1$-$C_6$-Alkyl oder durch Halogen, $C_1$-$C_3$-Alkoxy substituiertes $C_1$-$C_6$-Alkyl; $C_3$-$C_6$-Alkenyl oder durch Halogen substituiertes $C_3$-$C_6$-Alkenyl; Phenyl oder durch Halogen, $C_1$-$C_3$-Alkyl, $C_1$-$C_3$-Alkoxy oder Nitro substituiertes Phenyl bedeutet;
$R_5$ ferner einen Carbamoylrest CO-N(R''')R'' oder einen Oxycarbonylrest COOR'', worin R'' einen aliphatischen oder cycloaliphatischen Rest mit maximal 6 C-Atomen darstellt, der unsubstituiert oder durch Halogen substituiert ist, oder worin R'' einen Phenyl- oder Benzylrest bedeutet, der unsubstituiert oder im Ring durch Halogen, $C_1$-$C_3$-Alkyl, $C_1$-$C_3$-Alkoxy oder Nitro substituiert ist; und worin R''' Wasserstoff oder $C_1$-$C_4$-Alkyl bedeutet;
$R_6$ Wasserstoff, $C_1$-$C_8$-Alkyl oder $C_1$-$C_4$-Alkyl, das durch Halogen, Hydroxy, Cyano, $C_1$-$C_4$-Alkoxy, $C_1$-$C_4$-Alkylamino, Bis-($C_1$-$C_4$-Alkyl)-amino, $C_3$-$C_6$-Cycloalkyl, $COOC_1$-$C_3$-Alkyl oder durch Phenyl substituiert ist, wobei das Phenyl durch Halogen, $C_1$-$C_3$-Alkyl, $C_1$-$C_3$-Alkoxy oder Nitro substituiert sein kann;
$R_6$ ferner $C_3$-$C_6$-Cycloalkyl oder durch Methyl substituiertes $C_3$-$C_6$-Cycloalkyl; $C_3$-$C_6$-Alkenyl oder durch Halogen substituiertes $C_3$-$C_6$-Alkenyl; $C_3$-$C_6$-Alkinyl oder durch Halogen substituiertes $C_3$-$C_6$-Alkinyl;
$R_6$ ferner Phenyl oder durch Halogen, $C_1$-$C_3$-Alkyl, $C_1$-$C_3$-Alkoxy, $C_1$-$C_2$-Halogenalkyl, $C_1$-$C_2$-Halogenalkoxy oder Nitro substituiertes Phenyl; einen 5- oder 6-gliedrigen gesättigten oder ungesättigten heterocyclischen Rest mit ein bis 3 Heteroatomen, N, O oder S, wobei der heterocyclische Rest über ein $CH_2$-Brückenglied gebunden und durch Halogen, $C_1$-$C_3$-Alkyl, $C_1$-$C_3$-Alkoxy oder Nitro substituiert sein kann;
$R_6$ ferner die Gruppe $NH$-$CO$-$NH_2$, $NH$-$CS$-$NH_2$ oder $N(R_7)R_8$, worin $R_7$ Wasserstoff oder $C_1$-$C_3$-Alkyl und $R_8$ Wasserstoff, $C_1$-$C_3$-Alkyl, Phenyl oder durch Halogen, $C_1$-$C_3$-Alkyl, $C_1$-$C_3$-Alkoxy oder Nitro substituiertes Phenyl darstellen; unter Einschluss ihrer Säureadditionssalze oder Metallsalzkomplexe.

2. Verbindungen der Formel I gemäss Anspruch 1, in welcher bedeuten:
$R_1$ und $R_2$ unabhängig voneinander Wasserstoff, Halogen oder Methyl;
$R_3$ Methyl, Ethyl, Cyclopropyl oder durch Methyl oder Halogen ein- oder zweifach substituiertes Cyclopropyl;
$R_5$ Wasserstoff, $C_1$-$C_4$-Alkyl, durch Halogen, Hydroxy oder $C_1$-$C_2$-Alkoxy substituiertes $C_1$-$C_2$-Alkyl, Benzyl oder durch Halogen, Methyl oder Methoxy substituiertes Benzyl, $C_3$-$C_4$-Alkenyl, $C_3$-$C_4$-Alkinyl oder durch Halogen substituiertes $C_3$-$C_4$-Alkinyl;
$R_5$ ferner COR', worin R' $C_1$-$C_3$-Alkyl oder durch Halogen oder $C_1$-$C_2$-Alkoxy substituiertes $C_1$-$C_3$-Alkyl, Phenyl oder durch Halogen, Methyl oder Methoxy substituiertes Phenyl darstellt; oder COOR'', worin R'' $C_1$-$C_4$-Alkyl oder Phenyl bedeutet; oder CONHR'', worin R'' $C_1$-$C_4$-Alkyl oder Phenyl oder durch Halogen substituiertes Halogen darstellt;
$R_5$ ferner die Reste $CON(CH_3)_2$ oder $CON(CH_3)OCH_3$;
$R_6$ Wasserstoff, $C_1$-$C_4$-Alkyl, durch Halogen, Hydroxy, $C_1$-$C_2$-Alkoxy,$C_1$-$C_2$-Alkylamino, Bis-($C_1$-$C_2$-Alkyl)-amino, Cyclopropyl, Cyclohexyl, Benzyl oder p-Chlorbenzyl substituiertes $C_1$-$C_2$-Alkyl;
$R_6$ ferner Cyclopropyl, Cyclohexyl, Allyl, Chlorallyl oder Propargyl;
$R_6$ ferner Phenyl, durch Halogen, Methyl, Methoxy, Trifluormethyl oder Difluormethoxy substituiertes Phenyl; einen 5- oder 6-gliedrigen gesättigten oder ungesättigten heterocyclischen Rest mit ein bis drei Heteroatomen, N, O oder S, wobei der heterocyclische Rest über ein $CH_2$-Brückenglied gebunden und durch Halogen, Methyl, Methoxy oder Nitro substituiert sein kann;
$R_6$ ferner die Gruppe $NH$-$CO$-$NH_2$, $NH$-$CS$-$NH_2$ oder $N(R_7)R_8$, worin $R_7$ Wasserstoff oder Methyl und $R_8$ Wasserstoff, Methyl, Phenyl oder durch Halogen, Methyl, Methoxy substituiertes Phenyl bedeuten.

3. Verbindungen der Formel I gemäss Anspruch 1, in welcher bedeuten:
$R_1$ und $R_2$ unabhängig voneinander Wasserstoff oder Fluor,
$R_3$ Methyl, Ethyl, Cyclopropyl oder durch Methyl substituiertes Cyclopropyl;
$R_5$ Wasserstoff, Methyl, Ethyl, Hydroxyethyl, Halogenethyl, Benzyl oder durch Fluor, Chlor oder Methyl sub-

stituiertes Benzyl, $C_3$-$C_4$-Alkenyl, $C_3$-$C_4$-Alkinyl oder durch Halogen substituiertes $C_3$-$C_4$-Alkinyl;
$R_5$ ferner COR′, worin R′Methyl oder Ethyl oder durch Fluor, Chlor oder Brom substituiertes Methyl oder Ethyl; Phenyl oder durch Fluor, Chlor oder Methyl substituiertes Phenyl darstellt; oder COOR″, worin R″ Methyl, Ethyl oder CONHR″, worin R″ Methyl, Ethyl, Phenyl oder durch Chlor substituiertes Phenyl darstellt;
$R_5$ ferner die Reste $CON(CH_3)_2$ oder $CON(CH_3)OCH_3$;
$R_6$ Wasserstoff, Methyl, Ethyl, durch Chlor, Hydroxy, Methoxy, Methylamino, Dimethylamino, Cyclopropyl, Benzyl oder p-Chlorbenzyl substituiertes $C_1$-$C_2$-Alkyl;
$R_6$ ferner Cyclopropyl, Allyl, Chlorallyl oder Propargyl;
$R_6$ ferner Phenyl, durch Halogen, Methyl, Methoxy, Trifluormethyl oder Difluormethoxy substituiertes Phenyl; einen heterocyclischen Rest, z.B. Furan-2-yl, Thiophen-2-yl, Thiazol-2-yl, Pyridin-2-yl, Pyridin-3-yl, Pyridin-4-yl, Pyrimidin-2-yl, Pyrimidin-4-yl oder Pyrimidin-5-yl.
$R_6$ ferner die Gruppe $NH$-$CO$-$NH_2$ oder $N(R_7)R_8$, worin $R_7$ Wasserstoff oder Methyl und
$R_8$ Wasserstoff, Methyl, Phenyl oder durch Chlor oder Methyl substituiertes Phenyl bedeuten.

**4.** Verbindungen der Formel I gemäss Anspruch 1, in welcher bedeuten:
$R_1$ und $R_2$ Wasserstoff;
$R_3$ Methyl, Ethyl oder Cyclopropyl;
$R_5$ Wasserstoff, Methyl, Ethyl, Hydroxyethyl, Bromethyl, Chlorethyl, Benzyl oder durch Fluor, Chlor oder Methyl substituiertes Benzyl, Allyl, Propargyl oder Jodpropargyl;
$R_5$ ferner COR′, worin R′Methyl oder durch Chlor oder Brom substituiertes Methyl; Phenyl oder durch Fluor, Chlor oder Methyl substituiertes Phenyl darstellt; oder COOR″, oder CONHR″, worin R″ Methyl, Ethyl oder Phenyl bedeutet;
$R_6$ Wasserstoff, Methyl, Ethyl, durch Chlor, Hydroxy, Cyclopropyl oder Benzyl substituiertes $C_1$-$C_2$-Alkyl; oder Cyclopropyl, Cyclohexyl, Allyl oder Propargyl;
$R_6$ ferner Phenyl oder durch Fluor Chlor, Methyl oder Trifluormethyl substituiertes Phenyl; einen heterocyclischen Rest, z.B. Thiophen-2-yl, Thiazol-2-yl, Pyridin-2-yl, Pyridin-3-yl oder Pyrimidin-2-yl;
$R_6$ ferner die Gruppe $NH$-$CO$-$NH_2$ oder $NHR_8$, worin $R_8$ Wasserstoff, Methyl, Phenyl oder durch Chlor oder Methyl substituiertes Phenyl bedeuten.

**5.** Eine Verbindung der Formel I gemäss Anspruch 1 ausgewählt aus der Gruppe:
2-Anilino-4-formyloxim-6-methyl-pyrimidin (Verb. Nr. 1.1);
2-Anilino-4-formyl-O-methyloxim-6-methyl-pyrimidin (Verb. Nr. 1.4);
2-Anilino-4-formyloxim-6-ethyl-pyrimidin (Verb. Nr. 1.14);
2-Anilino-4-formyl-O-methyloxim-6-ethyl-pyrimidin (Verb. Nr. 1.45);
2-(3-Fluoranilino)-4-formyloxim-6-methyl-pyrimidin (Verb. Nr. 1.93);
2-(3-Fluoranilino)-4-formyl-O-methyloxim-6-methyl-pyrimidin (Verb. Nr. 1.89);
2-Anilino-4-formyl-(2-ethoxyethylimin)-6-cyclopropyl-pyrimidin (Verb. Nr. 2.1);
2-Anilino-4-formyl-(cyclopropylimin)-6-cyclopropyl-pyrimidin (Verb. Nr. 2.12).

**6.** Verfahren zur Herstellung der Verbindungen der Formel I, dadurch gekennzeichnet, dass man
1. ein Guanidinsalz der Formel IIa

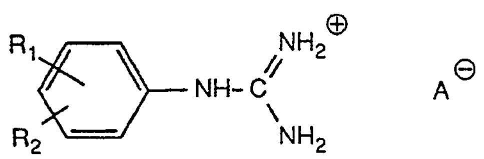

(IIa)

oder ein zugrundeliegendes Guanidin der Formel IIb

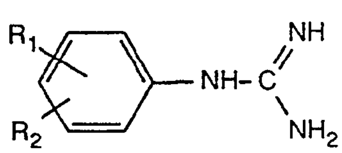

(IIb)

mit einem Diketon der Formel III,

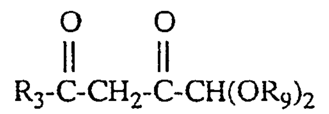

(III)

worin $R_9$ ein Alkyl, bevorzugt $C_1$-$C_4$-Alkyl, bedeutet, und $A^{\ominus}$ ein Säureanion darstellt, in einem protischen Lösungsmittel oder ohne Lösungsmittel bei Temperaturen von 40° bis 160°C, bevorzugt 60°C bis 110°C, zu einem Pyrimidinacetal der Formel IV

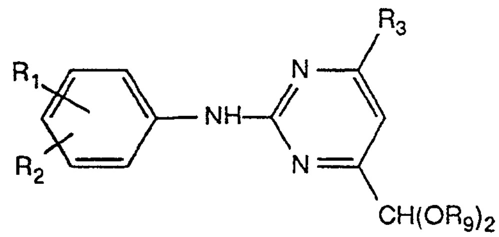

(IV)

umsetzt und das Acetal der Formel IV in Gegenwart einer Säure, z.B. Halogenwasserstoffsäure oder Schwefelsäure, in Wasser oder wässrigen Lösungsmittelgemischen, z.B. mit Lösungsmitteln wie Alkoholen oder Dimethylformamid, bei Temperaturen von 20° bis 100°C, bevorzugt 30°C bis 70°C, zu einem Pyrimidinaldehyd der Formel V

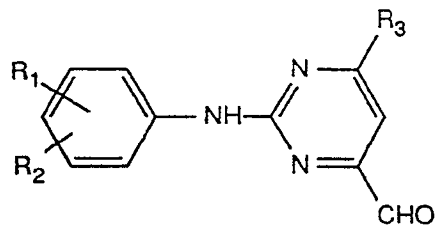

(V)

hydrolysiert und den Aldehyd der Formel V mit einem Hydroylaminsalz in Gegenwart einer Base in einem protischen Lösungsmittel, bevorzugt in Gegenwart von Wasser, bei Temperaturen von 10° bis 100°C, bevorzugt 10° bis 60°C, zu einem Oxim der Formel Ia

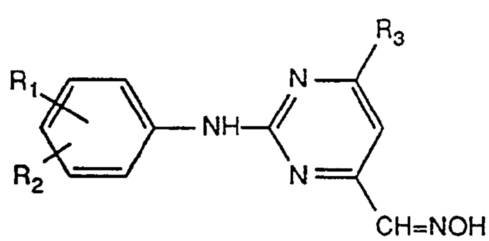

(Ia)

umsetzt, und das Oxim der Formel Ia mittels eines basischen Reagens, wie z.B. Natriumhydrid oder Kaliumhydroxyd in ein Oximsalz der Formel Ib

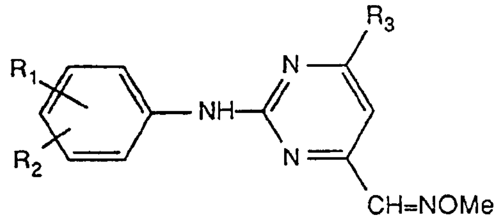

(Ib)

überführt, wobei Me ein Alkali oder Erdalkaliatom darstellt, und das Oximsalz der Formel (Ib) mit einer Verbindung der Formel VI

$R_5X$ (VI),

worin X eine nucleofuge Abgangsgruppe, wie z.B. Halogen, Mesylat, Tosylat u.a., vorzugsweise Chlor, Brom oder Jod, darstellt, in einem inerten Lösungsmittel bei Temperaturen von -30° bis 160°C, bevorzugt -10° bis 110°C, zu einer Verbindung der Formel Ic

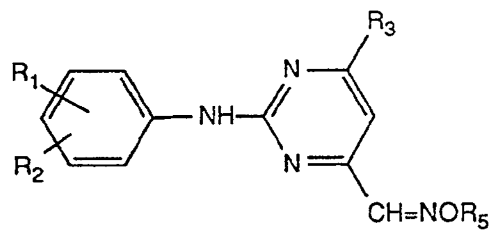

(Ic)

reagieren lässt; oder

2. ein Säurehalogenid der Formel VII, VIII oder IX

Hal-CO-R′ (VII)

Hal-CO-N(R″)R‴ (VIII)

Hal-CO-OR″ (IX)

oder ein analoges Säureanhydrid der Formel VII′, VIII′oder IX′

R′-CO-O-CO-R′ (VII′)

R‴(R″)N-CO-O-CO-N(R″)R‴ (VIII′)

R″O-CO-O-CO-OR″ (IX′)

mit einem Oxim der Formel Ia

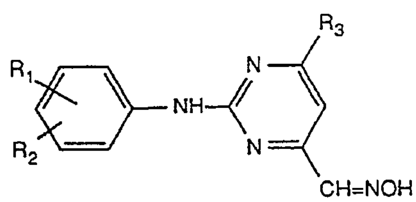

(Ia)

oder einem Salz davon in einem inerten Lösungsmittel und bei Verwendung der Säurehalogenide in Gegenwart von Protonenakzeptoren, z.B. tertiäre Amine, Alkali- oder Erdalkalihydroxide sowie Alkalihydride, bei Temperaturen von -20° bis 120°C, bevorzugt 0° bis 80°C, umsetzt, oder

3. ein Isocyanat der Formel X

R″N-CO (X)

mit einem Oxim der Formel Ia

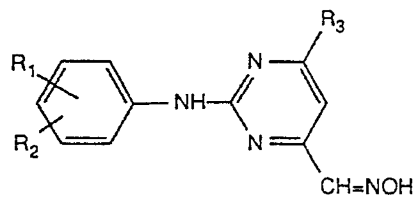

(Ia)

in einem inerten Lösungsmittel, wobei als Katalysator eine geringe Menge einer tertiären organischen Base, z.B. Triethylamin, zugeben sein kann, bei Temperaturen von 0° bis 120°C, bevorzugt 10° bis 80°C, umsetzt oder

4. eine Verbindung der Formel XI

$H_2N\text{-}OR_5\cdot HA$ (XI)

mit einem Formyldiacetal der Formel IV

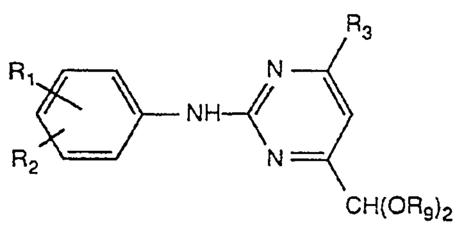

(IV)

oder einem Carbaldehyd der Formel V

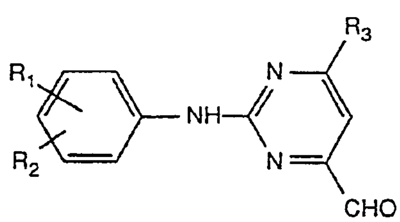

(V)

worin HA eine anorganische oder organische Säure, z.B. Schwefelsäure, Salzsäure, Essigsäure u.a., und $R_9$ ein Alkyl, bevorzugt $C_1$-$C_4$-Alkyl, darstellen, in einem inerten Lösungsmittel, z.B. einem protischen Lösungsmittel wie beispielsweise einem Alkohol, in Gegenwart einer Base (zur Freisetzung des Hydroxylamins) bei Temperaturen von 10° bis 110°C, bevorzugt 20° bis 80°C, umsetzt oder

5. ein Formyldiacetal der Formel IV

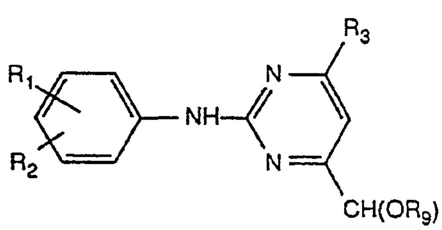

(IV)

oder ein Carbaldehyd der Formel V

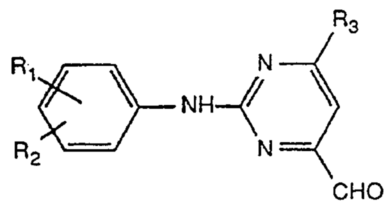

(V)

mit einem primären Amin der Formel XII

$H_2N\text{-}R_6$ (XII)

zu einer Verbindung der Formel Id

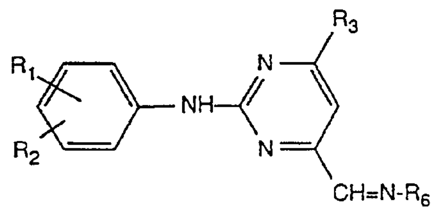

(Id)

worin $R_9$ ein Alkyl, bevorzugt $C_1$-$C_4$-Alkyl darstellt, in einem inerten Lösungsmittel, z.B Benzol, Toluol, Cyclohexan u.a., vorzugsweise mit einem Lösungsmittel, das mit dem ausgeschiedenen Wasser ein Azeotrop bildet, in Gegenwart eines Katalysators (zur Beschleunigung der Wasserabspaltung), z.B. von geringen Mengen anorganischer oder organischer Säuren, z.B. p-Toluolsulfonsäure, bei Temperaturen von 60° bis 160°C, bevorzugt 80° bis 120°C, umsetzt oder

6. ein Hydrazinderivat der Formel XIII, XIV oder XV

$H_2N\text{-}N(R_7)R_8$ (XIII)

$H_2N\text{-}NH\text{-}CO\text{-}NH_2$ (XIV)

$H_2N\text{-}NH\text{-}CS\text{-}NH_2$ (XV)

mit einem Formyldiacetal der Formel IV

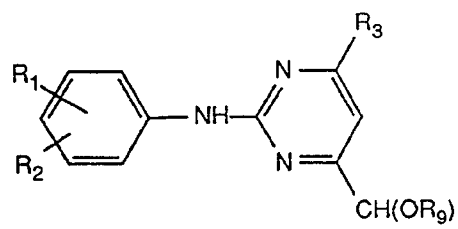

(IV)

oder einem Carbaldehyd der Formel V

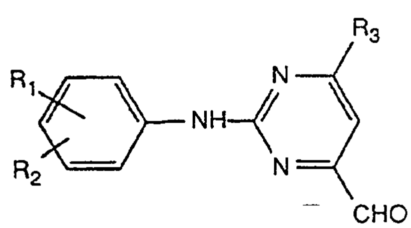

(V)

zu einer Verbindung der Formel Id'

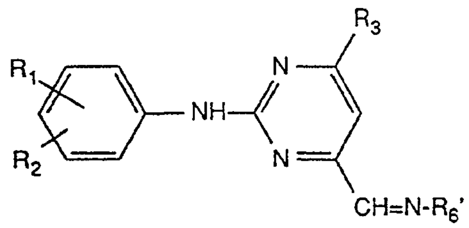

(Id')

worin $R_9$ ein Alkyl, bevorzugt $C_1$-$C_4$-Alkyl, und R6' die Reste NH-CO-$NH_2$, NH-CS-$NH_2$ oder $N(R_7)R_8$ darstellen, in einem inerten Lösungsmittel bei Temperaturen von -20° bis 120°C, bevorzugt 0° bis 80°C, umsetzt oder

7. eine Verbindung der Formel Id

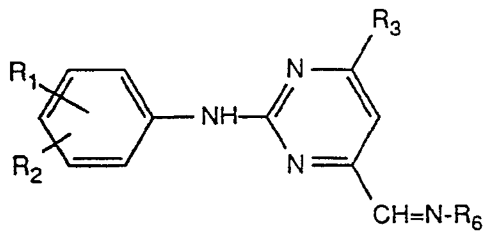

(Id)

durch Hydrierung mit elementarem Wasserstoff unter Normaldruck oder mit Ueberdruck in Gegenwart eines Katalysators, z.B. Palladium-Kohle oder Raney-Nickel in einem inerten Lösungsmittel, z.B. Ethylacetat, Tetrahydrofuran oder Dioxan, bei Temperaturen von 10° bis 120°C, bevorzugt 20° bis 60°C, in eine Verbindung der Formel Ie

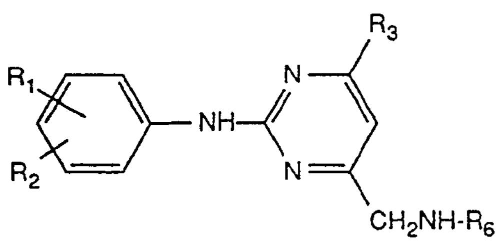

(Ie)

überführt; oder

8. eine Verbindung der Formel Ic

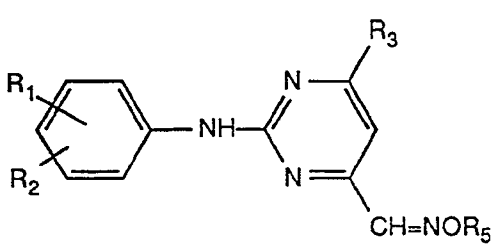

(Ic)

oder eine Verbindung der Formel Ig

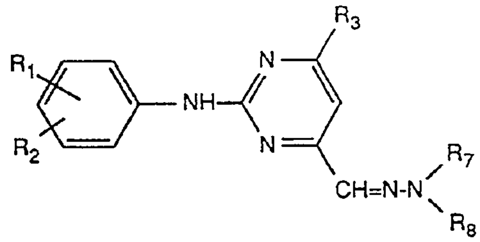

(Ig)

durch Hydrierung mit elementarem Wasserstoff unter Normaldruck oder mit Ueberdruck in Gegenwart eines Katalysators, z.B. Palladium-Kohle oder Raney-Nickel in einem inerten Lösungsmittel, z.B. Ethylacetat, Tetrahydrofuran oder Dioxan, bei Temperaturen von 10° bis 120°C, bevorzugt 20° bis 60°C, in eine Verbindung der Formel If

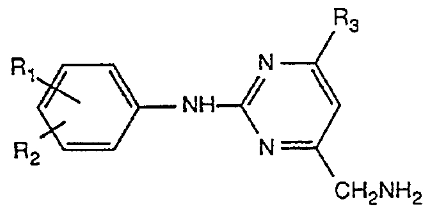

(If)

überführt, wobei in den vorstehenden Formeln Ia bis XVII $R_1$ bis $R_8$ die unter Formel I angegebenen Bedeutungen haben.

7. Mittel zum Schutz von Pflanzen gegen den Befall durch Mikroorganismen, dadurch gekennzeichnet, dass es als aktive Komponente mindestens eine Verbindung gemäss Anspruch 1 enthält.

8. Mittel gemäss Anspruch 7, dadurch gekennzeichnet, dass dieses zusätzlich mindestens noch einen Trägerstoff enthält.

9. Mittel gemäss Anspruch 7, dadurch gekennzeichnet, dass es als aktive Komponente mindestens eine Verbindung gemäss den Ansprüchen 2 bis 4 enthält.

10. Mittel gemäss Anspruch 7, dadurch gekennzeichnet, dass es als aktive Komponente eine Verbindung der Formel I gemäss Anspruch 5 enthält.

11. Verfahren zur Herstellung eines agrochemischen Mittels von Anspruch 7, dadurch gekennzeichnet, dass man mindestens eine gemäss Anspruch 1 definierte Verbindung mit geeigneten festen oder flüssigen Träger- und Hilfsstoffen innig vermischt.

12. Verwendung von Verbindungen gemäss Anspruch 1 zum Schutz von Pflanzen gegen den Befall durch phytopathogene Mikroorganismen.

13. Verwendung von Verbindungen gemäss einem der Ansprüche 2 bis 5 zum Schutz von Pflanzen gegen den Befall durch phytopathogene Mikroorganismen.

14. Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

15. Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 5 auf die Pflanze oder deren Standort appliziert.

16. Verfahren gemäss den Ansprüchen 14 und 15, dadurch gekennzeichnet, dass es sich bei den phytopathogenen Mikroorganismen um Pilzorganismen handelt.

17. Verbindungen der Formel V'

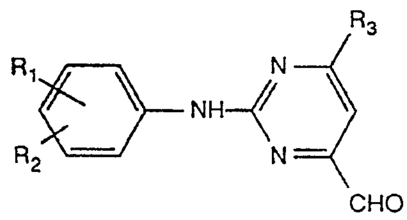

(V'),

in welcher bedeuten:
$R_1$ und $R_2$ unabhängig voneinander Wasserstoff, Halogen, $C_1$-$C_3$-Alkyl, $C_1$-$C_2$-Halogenalkyl, $C_1$-$C_3$-Alkoxy oder $C_1$-$C_3$-Halogenalkoxy:
$R_3$ $C_1$-$C_4$-Alkyl oder durch Halogen oder Hydroxy substituiertes $C_1$-$C_2$-Alkyl.

**Patentansprüche für folgenden Vertragsstaat: ES**

1. Verfahren zur Herstellung der Verbindungen der Formel I

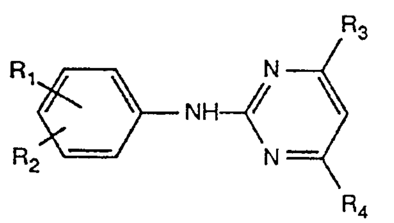

(I)

in welcher bedeuten:
$R_1$ und $R_2$ unabhängig voneinander Wasserstoff, Halogen, $C_1$-$C_3$-Alkyl, $C_1$-$C_2$-Halogenalkyl, $C_1$-$C_3$-Alkoxy oder $C_1$-$C_3$ Halogenalkoxy;
$R_3$ $C_1$-$C_4$-Alkyl, durch Halogen oder Hydroxy substituiertes $C_1$-$C_2$-Alkyl, $C_3$-$C_6$-Cycloalkyl oder durch Methyl oder Halogen bis zu dreifach gleich oder verschieden substituiertes $C_3$-$C_6$-Cycloalkyl;
$R_4$ die Reste CH=NOR$_5$, CH=NR$_6$ oder CH$_2$NHR$_6$;
$R_5$ Wasserstoff, $C_1$-$C_8$-Alkyl oder $C_1$-$C_4$-Alkyl, das durch Halogen, Hydroxy, Cyano, $C_1$-$C_4$-Alkoxy, COOC$_1$-$C_3$-Alkyl oder durch Phenyl substituiert ist, wobei das Phenyl durch Halogen, $C_1$-$C_3$-Alkyl, $C_1$-$C_3$-Alkoxy oder Nitro substituiert sein kann;
$R_5$ ferner $C_3$-$C_6$-Alkenyl oder durch Halogen substituiertes $C_3$-$C_6$-Alkenyl; $C_3$-$C_6$-Alkinyl oder durch Halogen substituiertes $C_3$-$C_6$-Alkinyl, oder einen Acylrest COR', worin R' $C_1$-$C_6$-Alkyl oder durch Halogen, $C_1$-$C_3$-Alkoxy substituiertes $C_1$-$C_6$-Alkyl; $C_3$-$C_6$-Alkenyl oder durch Halogen substituiertes $C_3$-$C_6$-Alkenyl; Phenyl oder durch Halogen, $C_1$-$C_3$-Alkyl, $C_1$-$C_3$-Alkoxy oder Nitro substituiertes Phenyl bedeutet;
$R_5$ ferner einen Carbamoylrest CO-N(R''')R'' oder einen Oxycarbonylrest COOR'', worin R'' einen aliphatischen oder cycloaliphatischen Rest mit maximal 6 C-Atomen darstellt, der unsubstituiert oder durch Halogen substituiert ist, oder worin R'' einen Phenyl- oder Benzylrest bedeutet, der unsubstituiert oder im Ring durch Halogen, $C_1$-$C_3$-Alkyl, $C_1$-$C_3$-Alkoxy oder Nitro substituiert ist; und worin R''' Wasserstoff oder $C_1$-$C_4$-Alkyl bedeutet;
$R_6$ Wasserstoff, $C_1$-$C_8$-Alkyl oder $C_1$-$C_4$-Alkyl, das durch Halogen, Hydroxy, Cyano, $C_1$-$C_4$-Alkoxy, $C_1$-$C_4$-Alkylamino, Bis-($C_1$-$C_4$-Alkyl)-amino, $C_3$-$C_6$-Cycloalkyl, COOC$_1$-$C_3$-Alkyl oder durch Phenyl substituiert ist, wobei das Phenyl durch Halogen, $C_1$-$C_3$-Alkyl, $C_1$-$C_3$-Alkoxy oder Nitro substituiert sein kann;
$R_6$ ferner $C_3$-$C_6$-Cycloalkyl oder durch Methyl substituiertes $C_3$-$C_6$-Cycloalkyl; $C_3$-$C_6$-Alkenyl oder durch

Halogen substituiertes $C_3$-$C_6$-Alkenyl; $C_3$-$C_6$-Alkinyl oder durch Halogen substituiertes $C_3$-$C_6$-Alkinyl; $R_6$ ferner Phenyl oder durch Halogen, $C_1$-$C_3$-Alkyl, $C_1$-$C_3$-Alkoxy, $C_1$-$C_2$-Halogenalkyl, $C_1$-$C_2$-Halogenalkoxy oder Nitro substituiertes Phenyl; einen 5- oder 6-gliedrigen gesättigten oder ungesättigten heterocyclischen Rest mit ein bis 3 Heteroatomen, N, O oder S, wobei der heterocyclische Rest über ein $CH_2$-Brückenglied gebunden und durch Halogen, $C_1$-$C_3$-Alkyl, $C_1$-$C_3$-Alkoxy oder Nitro substituiert sein kann;
$R_6$ ferner die Gruppe NH-CO-$NH_2$, NH-CS-$NH_2$ oder $N(R_7)R_8$, worin $R_7$ Wasserstoff oder $C_1$-$C_3$-Alkyl und $R_8$ Wasserstoff, $C_1$-$C_3$-Alkyl, Phenyl oder durch Halogen, $C_1$-$C_3$-Alkyl, $C_1$-$C_3$-Alkoxy oder Nitro substituiertes Phenyl darstellen; unter Einschluss ihrer Säureadditionssalze oder Metallsalzkomplexe; dadurch gekennzeichnet, dass man

1. ein Guanidinsalz der Formel IIa

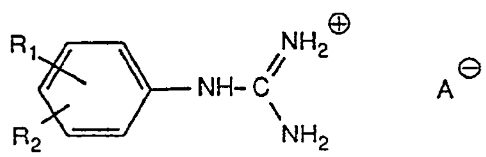

(IIa)

oder ein zugrundeliegendes Guanidin der Formel IIb

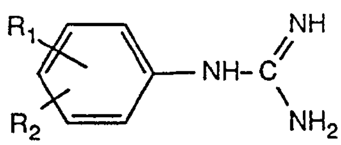

(IIb)

mit einem Diketon der Formel III,

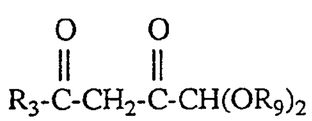

(III)

worin $R_9$ ein Alkyl, bevorzugt $C_1$-$C_4$-Alkyl, bedeutet, und $A^\ominus$ ein Säureanion darstellt, in einem protischen Lösungsmittel oder ohne Lösungsmittel bei Temperaturen von 40° bis 160°C, bevorzugt 60°C bis 110°C, zu einem Pyrimidinacetal der Formel IV

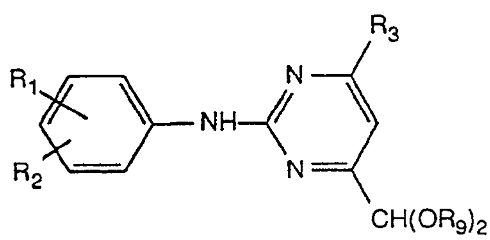

(IV)

umsetzt und das Acetal der Formel IV in Gegenwart einer Säure, z.B. Halogenwasserstoffsäure oder Schwefelsäure, in Wasser oder wässrigen Lösungsmittelgemischen, z.B. mit Lösungsmitteln wie Alkoholen oder Dimethylformamid, bei Temperaturen von 20° bis 100°C, bevorzugt 30°C bis 70°C, zu einem Pyrimidinaldehyd der Formel V

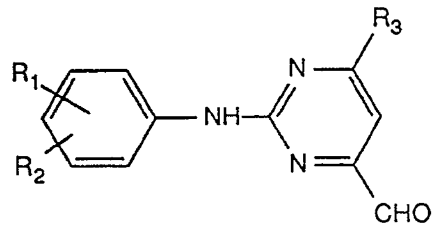

(V)

hydrolysiert und den Aldehyd der Formel V mit einem Hydroylaminsalz in Gegenwart einer Base in einem protischen Lösungsmittel, bevorzugt in Gegenwart von Wasser, bei Temperaturen von 10° bis 100°C, bevorzugt 10° bis 60°C, zu einem Oxim der Formel Ia

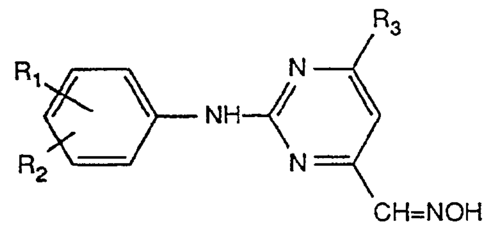

(Ia)

umsetzt, und das Oxim der Formel Ia mittels eines basischen Reagens, wie z.B. Natriumhydrid oder Kaliumhydroxyd in ein Oximsalz der Formel Ib

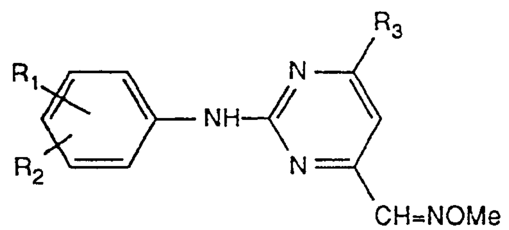

(Ib)

überführt, wobei Me ein Alkali oder Erdalkaliatom darstellt, und das Oximsalz der Formel (Ib) mit einer Verbindung der Formel VI

$R_5X$ (VI),

worin X eine nucleofuge Abgangsgruppe, wie z.B. Halogen, Mesylat, Tosylat u.a., vorzugsweise Chlor, Brom oder Jod, darstellt, in einem inerten Lösungsmittel bei Temperaturen von -30° bis 160°C, bevorzugt -10° bis 110°C, zu einer Verbindung der Formel Ic

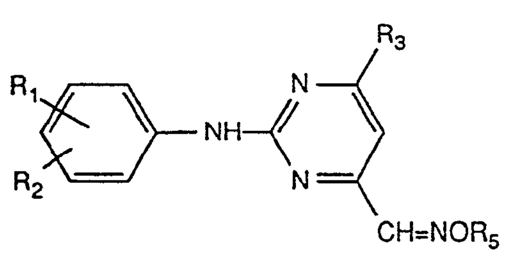

(Ic)

reagieren lässt; oder

2. ein Säurehalogenid der Formel VII, VIII oder IX

Hal-CO-R′ (VII)

Hal-CO-N(R″)R‴ (VIII)

Hal-CO-OR″ (IX)

oder ein analoges Säureanhydrid der Formel VII', VIII'oder IX'

R'-CO-O-CO-R' (VII')

R'''(R'')N-CO-O-CO-N(R'')R''' (VIII')

R''O-CO-O-CO-OR'' (IX')

mit einem Oxim der Formel Ia

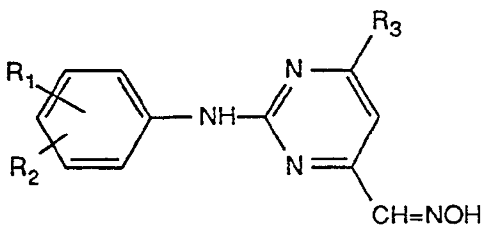

(Ia)

oder einem Salz davon in einem inerten Lösungsmittel und bei Verwendung der Säurehalogenide in Gegenwart von Protonenakzeptoren, z.B. tertiäre Amine, Alkali- oder Erdalkalihydroxide sowie Alkalihydride, bei Temperaturen von -20° bis 120°C, bevorzugt 0° bis 80°C, umsetzt, oder

3. ein Isocyanat der Formel X

R''N-CO (X)

mit einem Oxim der Formel Ia

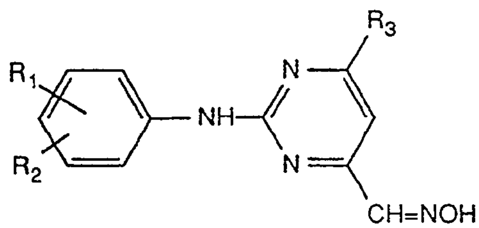

(Ia)

in einem inerten Lösungsmittel, wobei als Katalysator eine geringe Menge einer tertiären organischen Base, z.B. Triethylamin, zugegen sein kann, bei Temperaturen von 0° bis 120°C, bevorzugt 10° bis 80°C, umsetzt oder

4. eine Verbindung der Formel XI

$H_2N\text{-}OR_5\cdot HA$ (XI)

mit einem Formyldiacetal der Formel IV

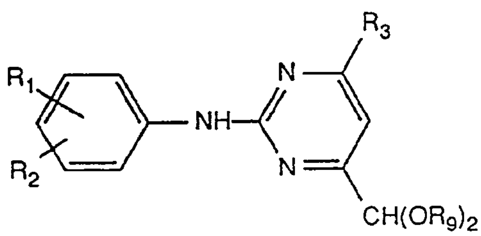

(IV)

oder einem Carbaldehyd der Formel V

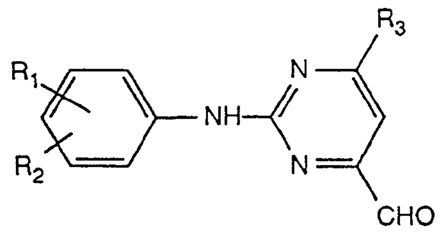

(V)

worin HA eine anorganische oder organische Säure, z.B. Schwefelsäure, Salzsäure, Essigsäure u.a., und $R_9$ ein Alkyl, bevorzugt $C_1$-$C_4$-Alkyl, darstellen, in einem inerten Lösungsmittel, z.B. einem protischen Lösungsmittel wie beispielsweise einem Alkohol, in Gegenwart einer Base (zur Freisetzung des Hydroxylamins) bei Temperaturen von 10° bis 110°C, bevorzugt 20° bis 80°C, umsetzt oder

5. ein Formyldiacetal der Formel IV

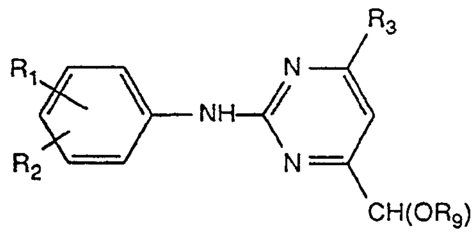

(IV)

oder ein Carbaldehyd der Formel V

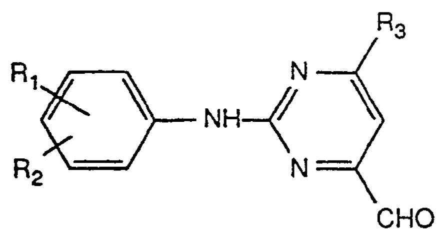

(V)

mit einem primären Amin der Formel XII

$H_2N$-$R_6$ (XII)

zu einer Verbindung der Formel Id

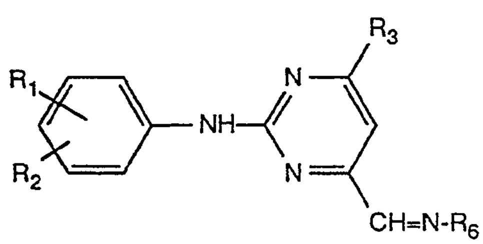

(Id)

worin $R_9$ ein Alkyl, bevorzugt $C_1$-$C_4$-Alkyl darstellt, in einem inerten Lösungsmittel, z.B. Benzol, Toluol, Cyclohexan u.a., vorzugsweise mit einem Lösungsmittel, das mit dem ausgeschiedenen Wasser ein Azeotrop bildet, in Gegenwart eines Katalysators (zur Beschleunigung der Wasserabspaltung), z.B. von geringen Mengen anorganischer oder organischer Säuren, z.B. p-Toluolsulfonsäure, bei Temperaturen von 60° bis 160°C, bevorzugt 80° bis 120°C, umsetzt oder

6. ein Hydrazinderivat der Formel XIII, XIV oder XV

$H_2N$-$N(R_7)R_8$ (XIII)

$H_2N-NH-CO-NH_2$ (XIV)
$H_2N-NH-CS-NH_2$ (XV)

mit einem Formyldiacetal der Formel IV

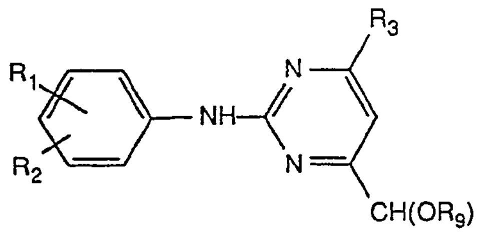

(IV)

oder einem Carbaldehyd der Formel V

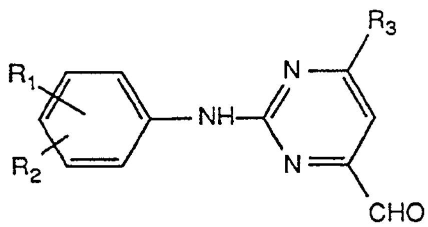

(V)

zu einer Verbindung der Formel Id'

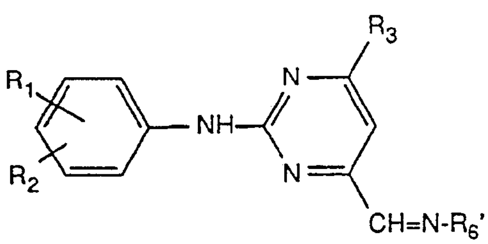

(Id')

worin $R_9$ ein Alkyl, bevorzugt $C_1$-$C_4$-Alkyl, und $R_6$'die Reste $NH-CO-NH_2$, $NH-CS-NH_2$ oder $N(R_7)R_8$ darstellen, in einem inerten Lösungsmittel bei Temperaturen von -20° bis 120°C, bevorzugt 0° bis 80°C, umsetzt oder

7. eine Verbindung der Formel Id

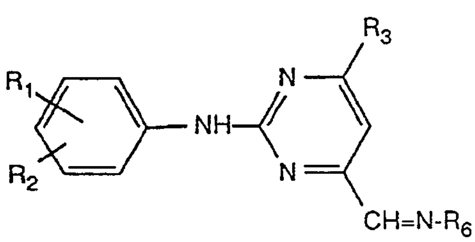

(Id)

durch Hydrierung mit elementarem Wasserstoff unter Normaldruck oder mit Ueberdruck in Gegenwart eines Katalysators, z.B. Palladium-Kohle oder Raney-Nickel in einem inerten Lösungsmittel, z.B. Ethylacetat, Tetrahydrofuran oder Dioxan, bei Temperaturen von 10° bis 120°C, bevorzugt 20° bis 60°C, in eine Verbindung der Formel Ie

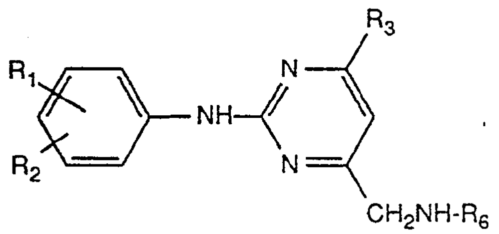

(Ie)

überführt; oder

8. eine Verbindung der Formel Ic

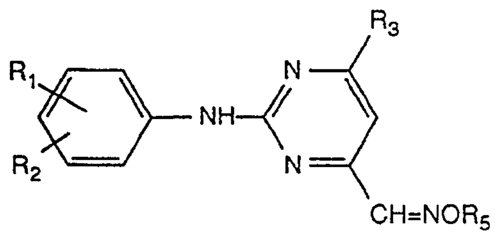

(Ic)

oder eine Verbindung der Formel Ig

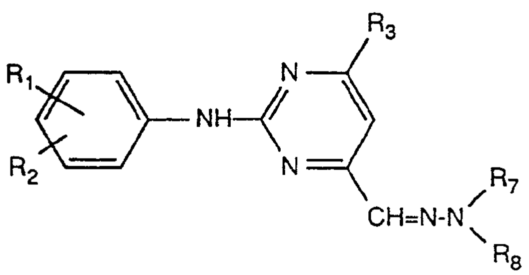

(Ig)

durch Hydrierung mit elementarem Wasserstoff unter Normaldruck oder mit Ueberdruck in Gegenwart eines Katalysators, z.B. Palladium-Kohle oder Raney-Nickel in einem inerten Lösungsmittel, z.B. Ethylacetat, Tetrahydrofuran oder Dioxan, bei Temperaturen von 10° bis 120°C, bevorzugt 20° bis 60°C, in eine Verbindung der Formel If

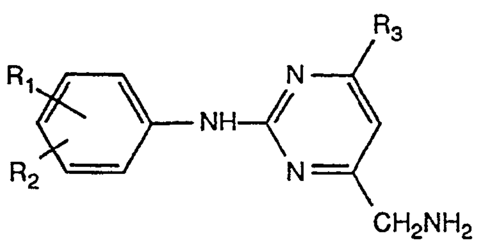

(If)

überführt, wobei in den vorstehenden Formeln Ia bis XVII $R_1$ bis $R_8$ die unter Formel I angegebenen Bedeutungen haben.

2. Verfahren gemäss Anspruch 1, dadurch gekennzeichnet, dass man Verbindungen der Formel I, in welcher bedeuten:

$R_1$ und $R_2$ unabhängig voneinander Wasserstoff, Halogen oder Methyl;

$R_3$ Methyl, Ethyl, Cyclopropyl oder durch Methyl oder Halogen ein- oder zweifach substituiertes Cyclopropyl;

$R_5$ Wasserstoff, $C_1$-$C_4$-Alkyl, durch Halogen, Hydroxy oder $C_1$-$C_2$-Alkoxy substituiertes $C_1$-$C_2$-Alkyl, Benzyl oder durch Halogen, Methyl oder Methoxy substituiertes Benzyl, $C_3$-$C_4$-Alkenyl, $C_3$-$C_4$-Alkinyl oder durch Halogen substituiertes $C_3$-$C_4$-Alkinyl;
$R_5$ ferner COR′, worin R′ $C_1$-$C_3$-Alkyl oder durch Halogen oder $C_1$-$C_2$-Alkoxy substituiertes $C_1$-$C_3$-Alkyl, Phenyl oder durch Halogen, Methyl oder Methoxy substituiertes Phenyl darstellt; oder COOR″, worin R″ $C_1$-$C_4$-Alkyl oder Phenyl bedeutet; oder CONHR″, worin R″ $C_1$-$C_4$-Alkyl oder Phenyl oder durch Halogen substituiertes Halogen darstellt;
$R_5$ ferner die Reste $CON(CH_3)_2$ oder $CON(CH_3)OCH_3$;
$R_6$ Wasserstoff, $C_1$-$C_4$-Alkyl, durch Halogen, Hydroxy, $C_1$-$C_2$ -Alkoxy, $C_1$-$C_2$-Alkylamino, Bis-($C_1$-$C_2$-Alkyl)-amino, Cyclopropyl, Cyclohexyl, Benzyl oder p-Chlorbenzyl substituiertes $C_1$-$C_2$-Alkyl;
$R_6$ ferner Cyclopropyl, Cyclohexyl, Allyl, Chlorallyl oder Propargyl;
$R_6$ ferner Phenyl, durch Halogen, Methyl, Methoxy, Trifluormethyl oder Difluormethoxy substituiertes Phenyl; einen 5- oder 6-gliedrigen gesättigten oder ungesättigten heterocyclischen Rest mit ein bis drei Heteroatomen, N, O oder S, wobei der heterocyclische Rest über ein $CH_2$-Brückenglied gebunden und durch Halogen, Methyl, Methoxy oder Nitro substituiert sein kann;
$R_6$ ferner die Gruppe NH-CO-$NH_2$, NH-CS-$NH_2$ oder $N(R_7)R_8$, worin $R_7$ Wasserstoff oder Methyl und $R_8$ Wasserstoff, Methyl, Phenyl oder durch Halogen, Methyl, Methoxy substituiertes Phenyl; herstellt.

3. Verfahren gemäss Anspruch 1, dadurch gekennzeichnet, dass man Verbindungen der Formel I, in welcher bedeuten:
$R_1$ und $R_2$ unabhängig voneinander Wasserstoff oder Fluor;
$R_3$ Methyl, Ethyl, Cyclopropyl oder durch Methyl substituiertes Cyclopropyl;
$R_5$ Wasserstoff, Methyl, Ethyl, Hydroxyethyl, Halogenethyl, Benzyl oder durch Fluor, Chlor oder Methyl substituiertes Benzyl, $C_3$-$C_4$-Alkenyl, $C_3$-$C_4$-Alkinyl oder durch Halogen substituiertes $C_3$-$C_4$-Alkinyl;
$R_5$ ferner COR′, worin R′Methyl oder Ethyl oder durch Fluor, Chlor oder Brom substituiertes Methyl oder Ethyl; Phenyl oder durch Fluor, Chlor oder Methyl substituiertes Phenyl darstellt; oder COOR″, worin R″ Methyl, Ethyl oder CONHR″, worin R″ Methyl, Ethyl, Phenyl oder durch Chlor substituiertes Phenyl darstellt;
$R_5$ ferner die Reste $CON(CH_3)_2$ oder $CON(CH_3)OCH_3$;
$R_6$ Wasserstoff, Methyl, Ethyl, durch Chlor, Hydroxy, Methoxy, Methylamino, Dimethylamino, Cyclopropyl, Benzyl oder p-Chlorbenzyl substituiertes $C_1$-$C_2$-Alkyl;
$R_6$ ferner Cyclopropyl, Allyl, Chlorallyl oder Propargyl;
$R_6$ ferner Phenyl, durch Halogen, Methyl, Methoxy, Trifluormethyl oder Difluormethoxy substituiertes Phenyl; einen heterocyclischen Rest, z.B. Furan-2-yl, Thiophen-2-yl, Thiazol-2-yl, Pyridin-2-yl, Pyridin-3-yl, Pyridin-4-yl, Pyrimidin-2-yl, Pyrimidin-4-yl oder Pyrimidin-5-yl.
$R_6$ ferner die Gruppe NH-CO-$NH_2$ oder $N(R_7)R_8$, worin $R_7$ Wasserstoff oder Methyl und
$R_8$ Wasserstoff, Methyl, Phenyl oder durch Chlor oder Methyl substituiertes Phenyl; herstellt.

4. Verfahren gemäss Anspruch 1, dadurch gekennzeichnet, dass man Verbindungen der Formel I, in welcher bedeuten:
$R_1$ und $R_2$ Wasserstoff;
$R_3$ Methyl, Ethyl oder Cyclopropyl;
$R_5$ Wasserstoff, Methyl, Ethyl, Hydroxyethyl, Bromethyl, Chlorethyl, Benzyl oder durch Fluor, Chlor oder Methyl substituiertes Benzyl, Allyl, Propargyl oder Jodpropargyl;
$R_5$ ferner COR′, worin R′Methyl oder durch Chlor oder Brom substituiertes Methyl; Phenyl oder durch Fluor, Chlor oder Methyl substituiertes Phenyl darstellt; oder COOR″, oder CONHR″, worin R″ Methyl, Ethyl oder Phenyl bedeutet;
$R_3$ Wasserstoff, Methyl, Ethyl, durch Chlor, Hydroxy, Cyclopropyl oder Benzyl substituiertes $C_1$-$C_2$-Alkyl; oder Cyclopropyl, Cyclohexyl, Allyl oder Propargyl;
$R_6$ ferner Phenyl oder durch Fluor Chlor, Methyl oder Trifluormethyl substituiertes Phenyl; einen heterocyclischen Rest, z.B. Thiophen-2-yl, Thiazol-2-yl, Pyridin-2-yl, Pyridin-3-yl oder Pyrimidin-2-yl;
$R_6$ ferner die Gruppe NH-CO-$NH_2$ oder $NHR_8$, worin $R_8$ Wasserstoff, Methyl, Phenyl oder durch Chlor oder Methyl substituiertes Phenyl; herstellt.

5. Verfahren gemäss Anspruch 1, dadurch gekennzeichnet, dass man eine Verbindung ausgewählt aus der Gruppe:
2-Anilino-4-formyloxim-6-methyl-pyrimidin (Verb. Nr. 1.1);
2-Anilino-4-formyl-O-methyloxim-6-methyl-pyrimidin (Verb. Nr. 1.4);
2-Anilino-4-formyloxim-6-ethyl-pyrimidin (Verb. Nr. 1.14);

2-Anilino-4-formyl-O-methyloxim-6-ethyl-pyrimidin (Verb. Nr. 1.45);
2-(3-Fluoranilino)-4-formyloxim-6-methyl-pyrimidin (Verb. Nr. 1.93);
2-(3-Fluoranilino)-4-formyl-O-methyloxim-6-methyl-pyrimidin (Verb. Nr. 1.89);
2-Anilino-4-formyl-(2-ethoxyethylimin)-6-cyclopropyl-pyrimidin (Verb. Nr. 2.1);
2-Anilino-4-formyl-(cyclopropylimin)-6-cyclopropyl-pyrimidin (Verb. Nr. 2.12)
herstellt.

6. Mittel zum Schutz von Pflanzen gegen den Befall durch Mikroorganismen, dadurch gekennzeichnet, dass es als aktive Komponente mindestens eine Verbindung von Anspruch 1 enthält.

7. Mittel gemäss Anspruch 6, dadurch gekennzeichnet, dass dieses zusätzlich mindestens noch einen Trägerstoff enthält.

8. Mittel gemäss Anspruch 6, dadurch gekennzeichnet, dass es als aktive Komponente mindestens eine Verbindung der Ansprüche 2 bis 4 enthält.

9. Mittel gemäss Anspruch 7, dadurch gekennzeichnet, dass es als aktive Komponente eine Verbindung der Formel I gemäss Anspruch 5 enthält.

Europäisches Patentamt

# EUROPÄISCHER RECHERCHENBERICHT

Nummer der Anmeldung

EP 91 81 0355

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | EP-A-270111 (KUMIAI CHEMICAL INDUSTRY CO.) * Seite 3, Zeile 45 - Seite 5 * | 1, 7 | C07D239/42 A01N43/54 |
| A | EP-A-264348 (CIBA-GEIGY AG) * Seiten 2 - 3, Zeile 22 * | 1, 7 | |
| A,D | EP-A-224339 (KUMIAI CHEMICAL INDUSTRY CO.) * Seiten 2 - 3 * | 1, 7 | |

RECHERCHIERTE SACHGEBIETE (Int. Cl.5 )

C07D239/00

Der vorliegende Recherchenbericht wurde für alle Patentansprüche erstellt

| Recherchenort | Abschlußdatum der Recherche | Prüfer |
|---|---|---|
| BERLIN | 14 AUGUST 1991 | KYRIAKAKOU, G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)